# EUROPEAN PATENT APPLICATION

(11) **EP 3 875 485 A1**
(43) Date of publication of application: **08.09.2021**
(21) Application number: 19878486.0
(22) Date of filing: 29.10.2019
(51) Int. Cl.: C07K 16/28, C07K 16/46, A61K 39/395, C12N 15/13

(54) **BISPECIFIC ANTIBODY BINDING TO CD20 AND CD3 AND USES THEREOF**

(30) Priority: 01.11.2018 CN 201811294887
(71) Applicant: Ampsource Biopharma Shanghai Inc., Shanghai 201318 (CN)
(72) Inventor: LI, Qiang, Shanghai 201318 (CN); JIA, Shixiang, Shanghai 201318 (CN); MA, Xinlu, Shanghai 201318 (CN); CUI, Xueyuan, Shanghai 201318 (CN); ZHANG, Yuhua, Shanghai 201318 (CN); CHEN, Si, Shanghai 201318 (CN)
(74) Representative: Prinz & Partner mbB
(86) International application number: PCT/CN2019/113930
(87) International publication number: WO 2020/088437

(57) **Abstract**

Disclosed is a bispecific antibody that specifically binds to surface antigens CD3 of immune cells and CD20 antigens on the surfaces of tumor cells, and that can bind to human CD3 with high affinity, inducing T cell proliferation, and mediating tumor cell killing. The bispecific antibody in an in vitro test was able to mediate the specific killing of target cells by T cells. The construction method thereof is simple, avoiding the possibility of mismatch between two sets of light chains and heavy chains of heterobispecific antibodies, thereby reducing the difficulty of antibody purification. The affinity of the obtained antibody is high, the side effects caused by cytokines are small, and safety is high.

## Description

### CROSS-REFERENCE TO RELATED APPLICATION(S)

This application claims priority to Chinese Patent Application No. 201811294887.4 filed Nov. 1, 2018, the disclosure of which is incorporated herein by reference in its entirety.

### TECHNICAL FIELD

The present disclosure relates to the field of immunology and, in particular, to a bispecific antibody against CD20 and CD3 and use thereof, especially use thereof for treating tumors.

### BACKGROUND

T lymphocytes play an important role in the process of cellular immunity. T cell-mediated cellular immunity is mainly that a T cell receptor (TCR) specifically recognizes an antigen peptide presented by a major histocompatibility complex (MHC) on the surface of cells so that intracellular signals of T cells are activated and target cells are specifically killed. This is very important for the timely clearance of abnormal cells in a body and the prevention of tumorigenesis. Tumors are developed as a result of immune escape due to the down-regulation or absence of MHC on the surface of most tumor cells.

T cell-engaging bispecific antibodies (TCBs) represent a very effective manner of redirecting activated cytotoxic T cells to tumors. CD3, which is expressed in mature T cells as a part of the T cell receptor, can transduce activation signals generated during antigen recognition by TCR. TCBs can simultaneously bind to tumor surface antigens and CD3ε subunits of the T cell receptor to establish physical links between T cells and tumor cells, so that T cells in a resting state are effectively activated to kill tumor cells, thereby achieving the effect of treating tumors (Smits N C, Sentman C L, Journal of Clinical Oncology, 2016: JCO 649970). TCBs activate T cells without relying on the traditional antigen presentation by MHC and double activation signals of costimulatory molecules. Therefore, TCBs eliminate the need for tumor-specific immune and overcome the dysfunction of T cells in a tumor microenvironment.

In recent years, to achieve correct assembly of two different semi-antibodies, scientists have designed and developed bispecific antibodies with various structures. These bispecific antibodies are generally divided into two categories. One category of bispecific antibodies contains no Fc region, such as BiTE, DART, TrandAbs and bi-Nanobody. Such bispecific antibodies have the advantages of a small molecular weight and an ability to be expressed in prokaryotic cells without concerning the problem of correct assembly. However, due to the absence of an Fc fragment and a relatively small molecular weight, these bispecific antibodies have relatively short half-lives. Moreover, bispecific antibodies in this form are easy to polymerize, have poor stability, and are expressed at a low level so that their clinical applications are limited. The other category of bispecific antibodies reserves an Fc domain, such as Triomabs, kih IgG, Cross-mab, orthoFab IgG, DVD IgG, IgG scFv and scFv₂-Fc. These bispecific antibodies form IgG-like structures with larger molecular structures and have longer half-lives due to cell endocytosis and recycling processes mediated by FcRn. Some or all of effector functions mediated by Fc are reserved, such as antibody-dependent cellular cytotoxicity (ADCC), complement-dependent cytotoxicity (CDC) and antibody-dependent cellular phagocytosis (ADCP). However, this category of bispecific antibodies cannot completely eliminate the production of mismatched products, either. Residual fractions of any mismatched molecules are difficult to separate from the products. Moreover, this method requires a lot of genetic engineering modifications such as mutations for two antibody sequences and is not simple and universal.

CD20 is a non-glycosylated type III transmembrane protein and mainly involved in mediating the proliferation and differentiation of B lymphocytes. As typical antigens on the surface of B cells in a human immune system, CD20 is only present on the surface of pre-B cells and mature B cells. It is expressed in more than 95% of B cell lymphomas, and not expressed in hematopoietic stem cells, plasma cells and other normal tissues. Therefore, CD20 can serve as a target for the study on B cell tumors and autoimmune diseases. At present, multiple CD20 monoclonal antibody drugs have been marketed for the treatment of non-Hodgkin's lymphoma (NHL), chronic lymphocytic leukemia (CLL) and rheumatoid arthritis (RA) with good effects. The main mechanisms of the CD20 monoclonal antibody drugs are mainly complement-dependent cytotoxicity reactions, antibody-dependent cellular cytotoxicity, and increased sensitivity to cytotoxicity so that tumor cells expressing CD20 are killed. Although anti-CD20 tumor targeting strategies have shown great potential in clinical applications, not all patients respond to anti-CD20 treatment, and some patients show resistance to anti-CD20 treatment (e.g., resistance to rituximab). Therefore, a bispecific antibody targeting CD20 and CD3 can enhance the killing effect of immune cells on tumor cells and be used for treating CD20-positive B cell malignancies, such as non-Hodgkin's lymphoma and chronic lymphocytic leukemia. The bispecific antibody targeting CD20 and CD3 has a broad application prospect in the field of tumor immunology.

Therefore, the present disclosure aims to develop a CD20 bispecific molecule with improved performance in terms of half-life, stability, safety and productibility.

### SUMMARY

An object of the present disclosure is to provide a tetravalent, homodimer-type bispecific antibody molecule targeting immune effector cell antigen CD3 and tumor antigen CD20. The bispecific antibody can significantly inhibit or kill tumor cells *in vivo* and has significantly reduced non-specific killing effect on normal cells with the low expression of CD20 and controlled toxic side effects due to the over-activation of effector cells and significantly improved physicochemical and *in vivo* stability.

Specifically, in a first aspect of the present disclosure, there is disclosed a bispecific antibody, which is a tetravalent homodimer formed by two identical polypeptide chains that bind each other by a covalent bond, wherein each of the polypeptide chains includes a first single-chain Fv that specifically binds to tumor antigen CD20, a second single-chain Fv that specifically binds to effector cell antigen CD3 and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions comprising CDC, ADCC and ADCP.

The first single-chain Fv has specificity to tumor-associated antigen CD20 and includes a VH domain and a VL domain linked by a linker peptide (L1), wherein VH, L1 and VL are arranged in order of VH-L1-VL or VL-L1-VH and the linker peptide L1 has an amino acid sequence of (GGGGX)ₙ, wherein X includes Ser or Ala, preferably Ser; and n is a natural number from 1 to 5, preferably 3;

For example, some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the first single-chain Fv against the tumor-associated antigen are exemplarily listed in Table 6-1 of the present disclosure.

In a preferred embodiment of the present disclosure, the amino acid sequence of the linker peptide L1 is (GGGGS)3. In other preferred embodiments, the amino acid sequence of the linker peptide L1 also includes (GGGGS)1, (GGGGS)2, (GGGGS)4, (GGGGS)5, (GGGGA)1, (GGGGA)2, (GGGGA)3, (GGGGA)4 or (GGGGA)5.

Preferably, the first single-chain Fv is selected from the group consisting of:
(i) a VH domain comprising HCDR1, HCDR2 and HCDR3 as shown in SEQ ID NOs: 1, 2 and 3, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 1, 2 and 3; and a VL domain comprising LCDR1, LCDR2 and LCDR3 as shown in SEQ ID NOs: 4, 5 and 6, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 4, 5 and 6;
(ii) a VH domain comprising HCDR1, HCDR2 and HCDR3 as shown in SEQ ID NOs: 7, 8 and 9, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 7, 8 and 9; and a VL domain comprising LCDR1, LCDR2 and LCDR3 as shown in SEQ ID NOs: 10, 11 and 12, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 10, 11 and 12;
(iii) a VH domain comprising HCDR1, HCDR2 and HCDR3 as shown in SEQ ID NOs: 13, 14 and 15, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 13, 14 and 15; and having VL domain comprising LCDR1, LCDR2 and LCDR3 as shown in SEQ ID NOs: 16, 17 and 18, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 16, 17 and 18; and
(iv) a VH domain comprising HCDR1, HCDR2 and HCDR3 as shown in SEQ ID NOs: 19, 20 and 21, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 19, 20 and 21; and a VL domain comprising LCDR1, LCDR2 and LCDR3 as shown in SEQ ID NOs: 22, 23 and 24, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 22, 23 and 24.

More preferably, the first single-chain Fv is selected from the group consisting of:
(i) a VH domain comprising an amino acid sequence as shown in SEQ ID NO: 25 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 25; and a VL domain comprising an amino acid sequence as shown in SEQ ID NO: 26 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 26;
(ii) a VH domain comprising an amino acid sequence as shown in SEQ ID NO: 27 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 27; and a VL domain comprising an amino acid sequence as shown in SEQ ID NO: 28 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 28;
(iii) a VH domain comprising an amino acid sequence as shown in SEQ ID NO: 29 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 29; and a VL domain comprising an amino acid sequence as shown in SEQ ID NO: 30 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 30; and
(iv) a VH domain comprising an amino acid sequence as shown in SEQ ID NO: 31 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 31; and a VL domain comprising an amino acid sequence as shown in SEQ ID NO: 32 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 32.

The linker peptide (L2) that links the first single-chain Fv and the second single-chain Fv of the present disclosure consists of a flexible peptide and a rigid peptide.

Further, the flexible peptide includes two or more amino acids, and preferably selected from the following amino acids: Gly(G), Ser(S), Ala(A) and Thr(T). More preferably, the flexible peptide includes G and S residues. Most preferably, an amino acid composition structure of the flexible peptide has a general formula of GₓS_{y}(GGGGS)_{z}, wherein x, y and z are integers greater than or equal to 0 and x + y + z ≥ 1. For example, in a preferred embodiment, the amino acid sequence of the flexible peptide is G₂(GGGGS)₃.

Further, the rigid peptide is derived from a full-length sequence (as shown in SEQ ID NO: 33) consisting of amino acids 118 to 145 at the carboxyl terminus of natural human chorionic gonadotropin β-subunit or a truncated fragment thereof (hereinafter collectively referred to as a CTP). Preferably, the CTP rigid peptide includes 10 amino acids at the N-terminus of SEQ ID NO: 33, that is, SSSSKAPPPS (CTP¹); or the CTP rigid peptide includes 14 amino acids at the C-terminus of SEQ ID NO: 33, that is, SRLPGPSDTPILPQ (CTP²); as another example, in another embodiment, the CTP rigid peptide includes 16 amino acids at the N-terminus of SEQ ID NO: 33, that is, SSSSKAPPPSLPSPSR (CTP³); as another example, in some other embodiments, the CTP rigid peptide includes 28 amino acids which begin at position 118 and end at position 145 of the human chorionic gonadotropin β-subunit, that is, SSSSKAPPPSLPSPSRLPGPSDTPILPQ (CTP⁴).

For example, some preferred amino acid sequences of the linker peptide L2 that links the first single-chain Fv and the second single-chain Fv are exemplarily listed in Table 3 of the present disclosure.

In a preferred embodiment of the present disclosure, the linker peptide L2 has an amino acid sequence as shown in SEQ ID NO: 52, wherein the amino acid composition of the flexible peptide thereof is G₂(GGGGS)₃, and the amino acid composition of the rigid peptide thereof is SSSSKAPPPS (i.e. CTP¹).

The second single-chain Fv has specificity to immune effector cell antigen CD3 and includes a VH domain and a VL domain linked by a linker peptide (L3), wherein VH, L3 and VL are arranged in order of VH-L3-VL or VL-L3-VH and the linker peptide L3 has an amino acid sequence of (GGGGX)ₙ, wherein X includes Ser or Ala, preferably Ser, and n is a natural number from 1 to 5, preferably 3.

Preferably, the second single-chain Fv of the bispecific antibody binds to effector cells at an EC₅₀ value greater than 50 nM, or greater than 100 nM, or greater than 300 nM, or greater than 500 nM in an *in vitro* FACS binding assay; more preferably, the second single-chain Fv of the bispecific antibody is capable of binding to human CD3 and specifically binding to CD3 of a cynomolgus monkey or a rhesus monkey. In a preferred embodiment of the present disclosure, the bispecific antibody specifically binds to effector cells at an EC₅₀ value of 132.3 nM.

For example, some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the anti-CD3 scFv are exemplarily listed in Table 2 of the present disclosure.

Preferably, the second single-chain Fv specifically binds to CD3; the VH domain of the second single-chain Fv contains HCDR1, HCDR2 and HCDR3 as shown in SEQ ID NOs: 34, 35 and 36, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 34, 35 and 36; and the VL domain of the second single-chain Fv contains LCDR1, LCDR2 and LCDR3 as shown in SEQ ID NOs: 37, 38 and 39, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 37, 38 and 39.

Preferably, the second single-chain Fv specifically binds to CD3; the VH domain of the second single-chain Fv contains HCDR1, HCDR2 and HCDR3 as shown in SEQ ID NOs: 40, 41 and 42, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 40, 41 and 42; and the VL domain of the second single-chain Fv contains LCDR1, LCDR2 and LCDR3 as shown in SEQ ID NOs: 43, 44 and 45, respectively or has sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 43, 44 and 45.

More preferably, the second single-chain Fv specifically binds to CD3; the VH domain of the second single-chain Fv contains an amino acid sequence as shown in SEQ ID NO: 46 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 46; and the VL domain of the second single-chain Fv contains an amino acid sequence as shown in SEQ ID NO: 47 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 47.

More preferably, the second single-chain Fv specifically binds to CD3; the VH domain of the second single-chain Fv contains an amino acid sequence as shown in SEQ ID NO: 48 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 48; and the VL domain of the second single-chain Fv contains an amino acid sequence as shown in SEQ ID NO: 49 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 49.

In a preferred embodiment of the present disclosure, the amino acid sequence of the linker peptide L3 is (GGGGS)3. In other preferred embodiments, the amino acid sequence of the linker peptide L3 also includes (GGGGS)1, (GGGGS)2, (GGGGS)4, (GGGGS)5, (GGGGA)1, (GGGGA)2, (GGGGA)3, (GGGGA)4 or (GGGGA)5.

The Fc fragment of the present disclosure is linked to the second single-chain Fv directly or by the linker peptide L4, and the linker peptide L4 includes 1-20 amino acids, and preferably selected from the following amino acids: Gly(G), Ser(S), Ala(A) and Thr(T); more preferably, the linker peptide L4 is selected from Gly (G) and Ser (S); further preferably, the linker peptide L4 consists of (GGGGS)n, wherein n = 1, 2, 3 or 4. In a preferred embodiment of the present disclosure, the Fc fragment is directly linked to the second single-chain Fv.

In another aspect, the Fc fragment of the present disclosure includes a hinge region, a CH2 domain and a CH3 domain from a human immunoglobulin heavy chain constant region. For example, in some embodiments, the Fc fragment of the present disclosure is selected from, for example, heavy chain constant regions of human IgG1, IgG2, IgG3, IgG4, IgM, IgA1, IgA2, IgD and IgE; particularly selected from heavy chain constant regions of human IgG1, IgG2, IgG3 and IgG4, and more particularly selected from a heavy chain constant region of human IgG1 or IgG4; and the Fc fragment has one or more amino acid substitutions, deletions or additions (for example, at most 20, at most 15, at most 10, or at most 5 substitutions, deletions, or additions) than a natural sequence from which the Fc fragment is derived.

In some preferred embodiments, the Fc fragment is changed, for example, mutated to modify the properties of the bispecific antibody molecule of the present disclosure (for example, to change one or more of the following properties: Fc receptor binding, antibody glycosylation, an effector cell function or a complement function).

For example, the bispecific antibody provided by the present disclosure includes an Fc variant containing amino acid substitutions, deletions or additions that change (for example, reduce or eliminate) effector functions. Fc region of the antibody mediates several important effector functions such as ADCC, ADCP and CDC. Methods for changing the affinity of the antibody to an effector ligand (such as FcyR or a complement C1q) by substituting amino acid residues in the Fc region of the antibody to change the effector functions are known in the art (see, for example, EP388151A1; US5648260; US5624821; Natsume A et al., Cancer Res., 68: 3863-3872, 2008; Idusogie EE et al., J. Immunol., 166: 2571-2575, 2001; Lazar GA et al., PNAS, 103: 4005-4010, 2006; Shields RL et al., JBC, 276: 6591-6604, 2001; Stavenhagen JB et al., Cancer Res., 67: 8882-8890, 2007; Stavenhagen JB et al., Advan. Enzyme. Regul., 48: 152-164, 2008; Alegre ML et al., J. Immunol., 148: 3461-3468, 1992; Kaneko E et al., Biodrugs, 25: 1-11, 2011). In some preferred embodiments of the present disclosure, amino acid L235 (EU numbering) in the constant region of the antibody is modified to change an interaction with an Fc receptor, such as L235E or L235A. In some other preferred embodiments, amino acids 234 and 235 in the constant region of the antibody are modified simultaneously, such as L234A and L235A (L234A/L235A) (EU numbering).

For example, the bispecific antibody provided by the present disclosure may include an Fc variant containing amino acid substitutions, deletions or additions that extend a circulating half-life. Studies show that M252Y/S254T/T256E, M428L/N434S or T250Q/M428L can extend the half-life of the antibody in primates. For more mutation sites included in the Fc variant with enhanced binding affinity to a neonatal receptor (FcRn), see Chinese invention patent CN 201280066663.2, US 2005/0014934A1, WO 97/43316, US 5,869,046, US 5,747,03 and WO 96/32478. In some preferred embodiments of the present disclosure, amino acid M428 (EU numbering) in the constant region of the antibody is modified to enhance the binding affinity to the FcRn receptor, such as M428L. In some other preferred embodiments, amino acids 250 and 428 (EU numbering) in the constant region of the antibody are modified simultaneously, such as T250Q and M428L (T250Q/M428L).

For example, the bispecific antibody provided by the present disclosure may also include an Fc variant containing amino acid substitutions, deletions or additions that can reduce or eliminate Fc glycosylation. For example, the Fc variant contains reduced glycosylation of the N-linked glycan normally present at amino acid site 297 (EU numbering). The glycosylation at position N297 has a great effect on the activity of IgG. If the glycosylation at this position is eliminated, the conformation of the upper half of CH2 of an IgG molecule is affected, thus losing the ability of binding to FcyRs and affecting the biological activity related to the antibody. In some preferred embodiments of the present disclosure, amino acid N297 (EU numbering) in the constant region of human IgG is modified to avoid the glycosylation of the antibody, such as N297A.

For example, the bispecific antibody provided by the present disclosure may also include an Fc variant containing amino acid substitutions, deletions or additions that eliminate charge heterogeneity. Various post-translational modifications during expression in engineered cells will cause the charge heterogeneity of monoclonal antibodies. The heterogeneity of lysine at C-terminus of an IgG antibody is one of the main reasons for charge heterogeneity. Lysine at C-terminus of a heavy chain may be deleted at a certain proportion during the production of the antibody, resulting in charge heterogeneity and affecting the stability, effectiveness, immunogenicity or pharmacokinetic of the antibody. In some preferred embodiments of the present disclosure, K447 (EU numbering) at the C-terminus of the IgG antibody is removed or deleted to eliminate the charge heterogeneity of the antibody and improve the homogeneity of the expressed product.

Some preferred amino acid sequences of the Fc fragment are exemplarily listed in Table 4 of the present disclosure. Compared with a bispecific antibody containing the Fc region of wild-type human IgG, the bispecific antibody provided by the present disclosure contains an Fc fragment that exhibits reduced affinity to at least one of human FcyRs (FcγRI, FcγRIIa, or FcγRIIIa) and C1q and has reduced effector cell functions or complement functions. For example, in a preferred embodiment of the present disclosure, the bispecific antibody includes an Fc fragment that is derived from human IgG1, has L234A and L235A substitutions (L234A/L235A), and exhibits reduced binding ability to FcγRI. In addition, the Fc fragment included in the bispecific antibody provided by the present disclosure may also contain amino acid substitutions that change one or more other characteristics (such as an ability of binding to the FcRn receptor, the glycosylation of the antibody or the charge heterogeneity of the antibody). For example, in a preferred embodiment of the present disclosure, the Fc fragment has an amino acid sequence as shown in SEQ ID NO: 57, which has amino acid substitutions L234A/L235A/T250Q/N297A/P331 S/M428L and a deleted or removed K447 compared with the natural sequence from which it is derived.

The bispecific antibody molecule of the present disclosure is a tetravalent homodimer formed by two identical polypeptide chains that bind to each other by an interchain disulfide bond in hinge regions of Fc fragments, and each polypeptide chain consists of an anti-CD20 scFv, a linker peptide, an anti-CD3 scFv and a Fc fragment in sequence from the N-terminus to the C-terminus.

In a preferred embodiment of the present disclosure, the bispecific antibody binds to human CD20 and CD3 and has an amino acid sequence as follows:
(1) a sequence as shown in SEQ ID NO: 50;
(2) a sequence having one or more substitutions, deletions or additions (such as 1, 2, 3, 4 or 5 substitutions, deletions or additions) relative to the sequence as shown in SEQ ID NO: 50; or
(3) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 50;

In some preferred embodiments, the substitutions in (2) are conservative substitutions.

In a second aspect of the present disclosure, there is provided a DNA molecule encoding the bispecific antibody as described above.

In a preferred embodiment of the present disclosure, the DNA molecule encoding the bispecific antibody as described above has a nucleotide sequence as shown in SEQ ID NO: 51.

In a third aspect of the present disclosure, there is provided a vector including the DNA molecule as described above.

In a fourth aspect of the present disclosure, there is provided a host cell including the vector as described above. The host cell includes a prokaryotic cell, a yeast or a mammalian cell. Preferably, the host cell is a mammalian cell such as a CHO cell, an NS0 cell or other mammalian cells. Further preferably, the host cell is a CHO cell.

In a fifth aspect of the present disclosure, there is provided a pharmaceutical composition including the bispecific antibody as described above and a pharmaceutically acceptable excipient, carrier or diluent.

In a sixth aspect of the present disclosure, there is provided a method for preparing the bispecific antibody of the present disclosure, including:
(a) obtaining a fusion gene of the bispecific antibody, and constructing an expression vector of the bispecific antibody;
(b) transfecting the expression vector into a host cell by a genetic engineering method;
(c) culturing the host cell under conditions that allow the bispecific antibody to be generated; and
(d) separating and purifying the bispecific antibody.

The expression vector in step (a) is one or more selected from a plasmid, a bacterium and a virus; preferably, the expression vector is a plasmid; more preferably, the expression vector is pCDNA3.1;

The host cell into which the constructed vector is transfected by a genetic engineering method in step (b) includes a prokaryotic cell, a yeast or a mammalian cell; preferably, the host cell is a mammalian cell such as a CHO cell, an NS0 cell or other mammalian cells; further preferably, the host cell is a CHO cell.

The bispecific antibody is separated and purified in step (d) by a conventional immunoglobulin purification method including protein A affinity chromatography and ion exchange, hydrophobic chromatography or molecular sieve.

In a seventh aspect of the present disclosure, there is provided use of the bispecific antibody for preparing a drug for treating, preventing or alleviating a tumor; Examples of the tumor include, but are not limited to, acute myeloid leukemia (AML), chronic myeloid leukemia (CML), B acute lymphocytic leukemia (B-ALL), B chronic lymphocytic leukemia (B-CLL), B-cell lymphoma (BCL), T-cell lymphoma (TCL) (such as skin), myelodysplastic syndrome (MDS), small lymphocytic lymphoma (SLL), hairy cell leukemia (HCL), marginal zone lymphoma (MZL) (such as extranodal or splenic), follicular lymphoma (FL) (such as pediatric or gastrointestinal), B-cell prolymphocytic leukemia (B-PLL), mantle cell lymphoma (MCL), lymphoplasmacytic lymphoma (LPL)/Waldenstrom's macroglobulinemia (WM), lymphoblastic leukemia (ALL) (such as B cell), lymphoblastic lymphoma (LBL) (such as B cell), plasmablastic lymphoma (PBL) (such as B cell), Hodgkin's lymphoma, non-Hodgkin's lymphoma, diffuse large B-cell lymphoma (DLBCL) (for example, primary or inflammation-related), Burkitt's lymphoma (BL), multiple myeloma, anaplastic large-cell lymphoma, HIV-related lymphoma and Waldenstrom's macroglobulinemia.

In an eighth aspect of the present disclosure, there is provided use of the bispecific antibody for preparing a drug for treating, preventing or alleviating an autoimmune or inflammatory disease, wherein the autoimmune or inflammatory disease is selected from rheumatoid arthritis (RA), osteoarthritis, reactive arthritis, systemic lupus erythematosus (SLE), Crohn's disease, multiple sclerosis, scleroderma, psoriasis, psoriatic arthritis, ulcerative colitis (such as chronic), insulin-dependent diabetes (such as juvenile), thyroiditis (such as chronic), hyperthyroidism, asthma, allergic diseases, sarcoidosis, autoimmune hemolytic anemia, pernicious anemia, graft-versus-host disease, dermatomyositis, chronic hepatitis, microscopic renal vasculitis, chronic active hepatitis, uveitis, intestinal synovitis, autoimmune intestinal disease, idiopathic leukopenia, autoimmune glomerulonephritis, autoimmune hemolytic anemia, autoimmune hepatitis, interstitial pneumonia, chronic pemphigus, pemphigus vulgaris, arteritis, polyarteritis nodosa and ankylosing spondylitis.

In a ninth aspect of the present disclosure, there is provided a method for enhancing or stimulating an immune response or function, comprising administering a therapeutically effective amount of the bispecific antibody or the pharmaceutical composition to an individual.

In a tenth aspect of the present disclosure, there is provided a method for preventing/treating, delaying development of, or reducing/inhibiting recurrence of a disease including diseases or disorders comprising an immune-related disease, a tumor, an autoimmune disease, an inflammatory disease or a transplant rejection-related disease or disorder, comprising administering an effective amount of the bispecific antibody or the pharmaceutical composition to an individual suffering from the diseases or disorders.

Preferably, the method may be used in combination with one or more other additional therapies, wherein the additional therapies are selected from the group consisting of a surgery, chemotherapy, radiotherapy, immunotherapy, gene therapy, DNA therapy, RNA therapy, nano therapy, viral therapy, adjuvant therapy and a combination thereof.

The technical solutions provided by the present disclosure have beneficial effects summarized as follows:
1. The bispecific antibody provided by the present disclosure includes anti-CD20 scFv that is located at the N terminus of the bispecific antibody and has changed spatial conformation, so that the bispecific antibody has reduced binding ability to CD20 under some conditions, especially that the bispecific antibody is difficult to bind to normal cells with weak expression or low expression of CD20, thereby exhibiting reduced non-specific killing effect. However, the binding specificity to cells with over-expression or high expression of CD20 is not significantly reduced, and the bispecific antibody exhibits a good killing effect *in vivo.* It can be known that when a target antigen is merely expressed on tumor cells or the bispecific antibody of the present disclosure specifically binds to only tumor cells over-expressing the target antigen, immune effector cells are activated restrictively and merely in target cell tissues, which can minimize the non-specific killing of the bispecific antibody on normal cells and the accompanying release of cytokines and reduce the toxic side effects of the bispecific antibody in clinical treatment.
2. The anti-CD3 scFv selected by the bispecific antibody provided by the present disclosure specifically binds to effector cells with weak binding affinity (EC₅₀ value greater than 50 nM, or greater than 100 nM, or greater than 300 nM, or greater than 500 nM). In addition, the anti-CD3 scFv is embedded between the anti-CD20 scFv and Fc, and the CTP rigid peptide contained in the linker peptide L3 at its N terminus and the Fc fragment located at its C terminus partially "cover" or "shield" the antigen binding domain of the anti-CD3 scFv. Such steric hindrance effect makes the anti-CD3 scFv bind to CD3 with weaker binding affinity (for example, greater than 1 µM), which reduces its ability to activate and stimulate T cells, limits the excessive release of cytokines, and provides higher safety. In addition, the anti-CD3 scFv used in the present disclosure can bind to CD3 natural antigens of human and a cynomolgus monkey and/or a rhesus monkey at the same time, so that no alternative molecule needs to be constructed for preclinical toxicology evaluation and the effective dose, toxic dose and toxic side effects obtained are more objective and accurate and can be directly converted into a clinical dose to reduce the risk of clinical studies.
3. The bispecific antibody provided by the present disclosure creatively adopts a divalent anti-CD3 scFv, which avoids the asymmetric structure of a heterodimer-type (including a monovalent anti-CD3 scFv) commonly used in the existing art in terms of the configuration design of the bispecific antibody and solves the problem of heavy chain mismatches, thereby simplifying downstream purification steps. Moreover, unexpectedly, non-specific binding of the anti-CD3 scFv to T cells is not observed in an *in vitro* cell binding assay, and the degree of cell activation (the release of cytokines such as IL-2) is controlled within a safe and effective range. That is, the bivalent anti-CD3 scFv structure used in the present disclosure has not induced over-activation of T cells in a non-antigen-dependent manner, whereas for other bispecific antibodies including bivalent anti-CD3 domains, the uncontrollable over-activation of T cells is common and thus anti-CD3 bispecific antibodies are generally designed to avoid the introduction of a bivalent anti-CD3 structure.
4. The modified Fc fragment included in the bispecific antibody provided by the present disclosure has no ability of binding to FcyR, avoiding the systemic activation of T cells mediated by FcyR and allowing immune effector cells to be activated restrictively and merely in target cell tissues.
5. The bispecific antibody provided by the present disclosure is homodimeric without mismatches of heavy chains and light chains. The bispecific antibody is produced by a stable downstream process and purified by simple and efficient steps, with a homogeneous expression product and significantly improved physicochemical and *in vivo* stability.

### Detailed description

Abbreviations and definitions
- BiAb: Bispecific antibody
- CDR: Complementarity determining region in a variable region of an immunoglobulin, defined by a Kabat numbering system
- EC₅₀: A concentration at which 50% efficacy or binding is generated
- ELISA: Enzyme-linked immunosorbent assay
- FR: Framework region of an antibody: a variable region of an immunoglobulin excluding CDRs
- HRP: Horseradish peroxidase
- IL-2: Interleukin 2
- IFN: Interferon
- IC₅₀: A concentration at which 50% inhibition is generated
- IgG: Immunoglobulin G
- Kabat: Immunoglobulin comparison and numbering system advocated by Elvin A Kabat
- mAb: Monoclonal antibody
- PCR: Polymerase chain reaction
- V region: IgG chain segment whose sequence is variable among different antibodies. It extends to Kabat residue 109 of the light chain and residue 113 of the heavy chain.
- VH: Heavy chain variable region of an immunoglobulin
- VK: κ light chain variable region of an immunoglobulin
- K_{D}: Equilibrium dissociation constant
- kₐ: Association rate constant
- k_{d}: Dissociation rate constant

In the present disclosure, unless otherwise specified, the scientific and technical terms used herein have meanings generally understood by those skilled in the art. The antibody or fragments thereof used in the present disclosure may be further modified using conventional techniques known in the art alone or in combination, such as amino acid deletion, insertion, substitution, addition, and/or recombination and/or other modification methods. A method for introducing such modifications into a DNA sequence of an antibody according to the amino acid sequence of the antibody is well known to those skilled in the art. See, for example, Sambrook, Molecular cloning: a laboratory manual, Cold Spring Harbor Laboratory (1989) N.Y.. Such modifications are preferably performed at a nucleic acid level. Meanwhile, for a better understanding of the present disclosure, the definitions and explanations of related terms are provided below.

"CD20", a specific marker molecule on the surface of B lymphocytes, is expressed in more than 90% of B lymphoma cells and normal B lymphocytes and not expressed in hematopoietic stem cells, primary B lymphocytes, normal blood cells and other tissues. CD20 is not significantly internalized and shed off and no antigen modulation is occurred upon binding to an antibody. CD20 may be used as an ideal target for the treatment of B-cell lymphoma. CD20 mainly exerts an anti-tumor effect through ADCC and CDC. In recent years, indications for the target CD20 have been increasingly expanded, which include, for example, autoimmune diseases (including multiple sclerosis, Crohn's disease) and inflammatory diseases (e.g., ulcerative colitis), etc. The indications for the target CD20 further include other related diseases or disorders that are found in the existing art and will be found in the future. The term also includes any variants, isoforms, derivatives and species homologues of CD20 that are naturally expressed by cells including tumor cells or expressed by cells transfected with CD20 genes or cDNA.

CD3 molecule is an important differentiation antigen on the T cell membrane and a characteristic marker of mature T cells. It is composed of six peptide chains, and these chains are associated with the T cell antigen receptor (TCR) with a non-covalent bond to constitute a TCR-CD3 complex. CD3 molecule not only participates in the intracytoplasmic assembly of the TCR-CD3 complex but also transmits antigen stimulation signals through the immunoreceptor tyrosine-based activation motif (ITAM) of the cytoplasmic regions of polypeptide chains. The main functions of CD3 molecule are to stabilize TCR structure and transmit T cell activation signal. When TCR specifically recognizes and binds to the antigen, CD3 is involved in signal transduction into T cell cytoplasm as the first signal to induce T cell activation and plays a very important role in T cell antigen recognition and immune response generation.

"CD3" refers to a part of a T-cell receptor complex and consists of three different chains CD3ε, CD3δ and CD3γ. CD3 is clustered on T cells by, for example, being immobilized by an anti-CD3 antibody, leading to the activation of T cells, which is similar to T cell receptor-mediated activation but independent of the specificity of TCR clones. Most anti-CD3 antibodies recognize the chain CD3ε. The second functional domain that specifically recognizes the T cell surface receptor CD3 in the present disclosure is not specifically limited as long as it can specifically recognize CD3, for example, but not limited to, CD3 antigens mentioned in the following patents: U.S. 7,994,289; U.S. 6,750,325; U.S. 6,706,265; U.S. 5,968,509; U.S. 8,076,459; U.S. 7,728,114; and U.S. 20100183615. Preferably, the antibody against human CD3 used in the present disclosure is cross-reactive with cynomolgus monkeys and/or rhesus monkeys, for example, but not limited to, CD3 antigens mentioned in the following patents: WO 2016130726, U.S. 20050176028, WO 2007042261, or WO 2008119565. The term also includes any variants, isotypes, derivatives, and species homologues of CD3 that are naturally expressed by cells or expressed by cells transfected with a gene or cDNA encoding the preceding chains.

The term "antibody" specifically includes a monoclonal antibody, a polyclonal antibody and an antibody-like polypeptide, such as a chimeric antibody and a humanized antibody. An "antigen binding fragment" includes fragments provided by any known technique such as enzymatic cleavage, peptide synthesis and recombination techniques. Some antigen binding fragments consist of intact antibody moieties that retain the antigen binding specificity of a parent antibody molecule. For example, the antigen binding fragment may include at least one variable region (heavy or light chain variable region) or one or more CDRs of an antibody that is known to bind to a particular antigen. Suitable examples of antigen binding fragments include, but are not limited to, a bispecific antibody, a single-chain molecule, an Fab molecule, an F(ab')2 molecule, an Fc molecule, an Fabc molecule, an Fv molecule, a single-chain (Sc) antibody, a individual antibody light chain, a individual antibody heavy chain, a chimeric fusion between an antibody chain or CDR and other proteins, a protein scaffold, a heavy chain monomer or dimer, a light chain monomer or dimer, a dimer consisting of one heavy chain and one light chain, a monovalent fragment consisting of VL, VH, CL and CH1 domains, or a monovalent antibody as described in WO 2007059782, a divalent fragment comprising two Fab fragments linked by a disulfide bond in the hinge region, and an Fd fragment consisting essentially of VH and CH1 domains; an Fv fragment consisting essentially of VL and VH domains of a single antibody arm, a dAb fragment (Ward et al., Nature, 1989, 341: 544-54) consisting essentially of a VH domain and also known as a domain antibody (Holt et al., Trends Biotechnol. 2003, 21 (11): 484 -90); a nanobody (Revets et al., Expert Opin Biol Ther. 2005 Jan; 5 (I): 111-24); or an isolated complementarity determining region (CDR), etc. All antibody isotypes may be used for producing antigen binding fragments. Additionally, the antigen binding fragment may include a non-antibody protein framework that can successfully incorporate a polypeptide fragment in an orientation that imparts affinity to a given antigen of interest (such as a protein scaffold). The antigen binding fragment may be recombinantly produced or produced through enzymatic or chemical cleavage of an intact antibody. The term "antibody or an antigen binding fragment thereof" may be used for representing that a given antigen binding fragment incorporates one or more amino acid fragments of the antibody referred to in the phrase.

The term "hypervariable region", "CDR" or "complementarity determining region" refers to amino acid residues of an antibody, which are responsible for antigen binding, and is a discontinuous amino acid sequence. CDR sequences are amino acid residues in the variable region that may be defined by the IMGT, Kabat, Chothia or AbM method or identified by any CDR sequence determination method well known in the art. For example, the hypervariable region includes the following amino acid residues: amino acid residues from a "complementarity determining region" or "CDR" defined by sequence comparison, for example, residues at positions 24-34 (L1), 50-56 (L2) and 89-97 (L3) in a light chain variable domain and residues at positions 31-35 (H1), 50-65 (H2) and 95-102 (H3) in a heavy chain variable domain (see Kabat et al., 1991, Sequences of Proteins of Immunological Interest (5th edition), Public Health Service, National Institutes of Health, Bethesda, Md.), and/or amino acid residues from a "hypervariable loop" (HVL) defined according to the structure, for example, residues at positions 26-32 (L1), 50-52 (L2) and 91-96 (L3) in the light chain variable domain and residues at positions 26-32 (H1), 53-55 (H2) and 96-101 (H3) in the heavy chain variable domain (see Chothia and Leskl, J. Mol Biol, 196: 901-917, 1987). "Framework" residues or "FR" residues refer to variable domain residues other than the hypervariable region residues as defined in the present disclosure. In some embodiments, the antibody or the antigen-binding fragment thereof in the present disclosure is preferably determined through the Kabat, Chothia or IMGT numbering system. Those skilled in the art may explicitly assign each system to any variable domain sequence without relying on any experimental data beyond the sequence itself. For example, the Kabat residue numbering method of a given antibody may be determined by comparing the sequence of the given antibody to each "standard" numbered sequence. Based on the numbers of the sequences provided herein, the numbering scheme of determining any variable region sequence in the sequence table is entirely within the conventional technical scope of those skilled in the art.

The term "single-chain Fv antibody" (or "scFv antibody") refers to an antibody fragment comprising VH and VL domains of an antibody. It is a fusion protein of the variable regions of the heavy (VH) and light chains (VL) connected with a linker. The linker enables these two domains to be cross-linked to form an antigen-binding site, and the sequence of the linker generally consists of a flexible peptide, for example, but not limited to, G₂(GGGGS)₃. The size of scFv is generally 1/6 of an intact antibody. The single-chain antibody is preferably an amino acid chain sequence encoded by a nucleotide chain. For the review of scFv, reference may be made to Pluckthun (1994), The Pharmacology of Monoclonal Antibodies, Vol. 113, edited by Rosenburg and Moore, Springer-Verlag, New York, pages 269-315. Reference may also be made to International Patent Application Publication No. WO 88/01649 and U.S. Patent Nos. 4,946,778 and 5,260,203.

The term "Fab fragment" consists of a light chain and a CH1 and a variable domain of each of a heavy chain. The heavy chain of the Fab molecule cannot form a disulfide bond with another heavy chain molecule. The size of "Fab antibody" is 1/3 of an intact antibody, and "Fab antibody" includes only one antigen-binding site.

The term "Fab' fragment" contains a light chain, and a VH domain and a CH1 domain of a heavy chain, and a constant region between CH1 and CH2 domains.

The term "F(ab')₂ fragment" contains two light chains, VH domains and CH1 domains of two heavy chains, and constant regions between CH1 and CH2 domains, so that an inter-chain disulfide bond is formed between the two heavy chains. Therefore, the F(ab')₂ fragment is composed of two Fab' fragments held together by the disulfide bond between the two heavy chains.

The term "Fc" region refers to the antibody heavy chain constant region fragment, including at least a hinge region and CH2 and CH3 domains.

The term "Fv region" includes variable regions from the heavy chain and the light chain but lacks the constant regions, and is the minimum fragment containing a complete antigen recognition and binding site.

The term "Fd fragment" consists of CH1 and variable regions of a heavy chain and is the heavy chain part of a Fab fragment excluding the light chain.

The term "linker peptide" refers to a peptide linking two polypeptides, wherein the linker peptide may be two immunoglobulin variable regions or one variable region. The length of the linker peptide may be 0 to 30 amino acids or 0 to 40 amino acids. In some embodiments, the linker peptide may be in the length of 0 to 25, 0 to 20, or 0 to 18 amino acids. In some embodiments, the linker peptide may be a peptide having no more than 14, 13, 12, 11, 10, 9, 8, 7, 6, or 5 amino acids. In other embodiments, the linker peptide may include 0 to 25, 5 to 15, 10 to 20, 15 to 20, 20 to 30, or 30 to 40 amino acids. In other embodiments, the linker peptide may have about 0, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids. The linker peptide is known to those skilled in the art. The linker peptide may be prepared by any method in the art. For example, the linker peptide may be originated from synthesis.

The term "heavy chain constant region" includes an amino acid sequence from the immunoglobulin heavy chain. The polypeptide comprising the heavy chain constant region includes at least one of: a CH1 domain, a hinge domain (e.g., an upper hinge region, an intermediate hinge region, and/or a lower hinge region), a CH2 domain, a CH3 domain, or a variant or fragment thereof. For example, the antigen-binding polypeptide used herein may include a polypeptide chain having a CH1 domain; a polypeptide having a CH1 domain, at least part of a hinge domain, and a CH2 domain; a polypeptide chain having a CH1 domain and a CH3 domain; a polypeptide chain having a CH1 domain, at least part of a hinge domain, and a CH3 domain; or a polypeptide chain having a CH1 domain, at least part of a hinge domain, a CH2 domain, and a CH3 domain. In another embodiment, the polypeptide of the present application includes a polypeptide chain having a CH3 domain. In addition, the antibody used in the present application may lack at least part of a CH2 domain (e.g., all or part of a CH2 domain). As described above, it is appreciated by those of ordinary skill in the art that heavy chain constant regions may be modified such that they differ in amino acid sequence from naturally immunoglobulin molecules.

The term "light chain constant region" includes an amino acid sequence from the antibody light chain. Preferably, the light chain constant region includes at least one of a constant kappa domain and a constant lambda domain.

The term "VH domain" includes an amino-terminal variable domain of the immunoglobulin heavy chain, while the term "CH1 domain" includes a first (mostly amino-terminal) constant region of the immunoglobulin heavy chain. The CH1 domain is adjacent to the VH domain and is the amino-terminal of the hinge region of the immunoglobulin heavy chain molecule.

The term "hinge region" includes the portion of a heavy chain molecule that links the CH1 domain to the CH2 domain. The hinge region contains about 25 residues and is flexible so that two N-terminal antigen binding regions move independently. The hinge region may be divided into three different domains: upper, middle and lower hinge domains (Roux KH et al., J. Immunol., 161: 4083, 1998).

The term "disulfide bond" includes a covalent bond formed between two sulfur atoms. The amino acid cysteine contains a sulfhydryl group which can form a disulfide bond or a bridge with a second sulfhydryl group. In most naturally occurring IgG molecules, CH1 and CK regions are linked by a disulfide bond and two heavy chains are linked by two disulfide bonds at positions 239 and 242 according to the Kabat numbering system (position 226 or 229, EU numbering system).

"Binding" is defined as an affinity interaction between a particular epitope on an antigen and its corresponding antibody and generally understood as "specific recognition". "Specific recognition" means that the bispecific antibody of the present disclosure does not cross-react with or substantially does not cross-react with any polypeptide other than the target antigen. The degree of specificity may be determined by immunological techniques, including, but not limited to, immunoblotting, immunoaffinity chromatography and flow cytometry, etc. In the present disclosure, the specific recognition is preferably determined through flow cytometry. In specific cases, the criteria for the specific recognition may be determined by those having ordinary skill in the art according to the common knowledge in the art.

The term "bispecific antibody" refers to the bispecific antibody of the present disclosure, for example, an anti-Her2 antibody or an antigen-binding fragment thereof, which may be derivatized or linked to another functional molecule, for example, another peptide or protein (e.g., TAA, a cytokine and a cell surface receptor), to generate a bispecific antibody that binds to at least two different binding sites or target molecules. To produce the bispecific molecule of the present disclosure, the antibody of the present disclosure may be functionally linked (e.g., by chemical coupling, gene fusion, non-covalent binding or other means) to one or more other binding molecules, for example, another antibody, antibody fragment, peptide or binding mimetic, to produce the bispecific molecule. For example, the "bispecific antibody" refers to one including two variable domains or ScFv units such that the antibody obtained recognizes two different antigens. Various different forms and uses of the bispecific antibody are known in the art (Chames P et al., Curr. Opin. Drug Disc. Dev., 12:.276, 2009; Spiess C et al., Mol. Immunol., 67: 95-106, 2015).

The term "hCG-β carboxy terminal peptide (CTP)" is a short peptide from the carboxy terminus of a human chorionic gonadotropin (hCG) β-subunit. Four reproduction-related polypeptide hormones, follicle-stimulating hormone (FSH), luteinizing hormone (LH), thyroid-stimulating hormone (TSH), and human chorionic gonadotropin (hCG), each contain the same α-subunit and their respective specific β-subunits. The *in vivo* half-life of hCG is significantly longer than those of the other three hormones, mainly due to the specific carboxy terminal peptide (CTP) on the β-subunit of hCG. The CTP includes 37 amino acid residues and four O-glycosylation sites, in which sugar side chain terminals are sialic acid residues. The electronegative highly-sialyl CTP can resist renal clearance and thus extend the half-life *in vivo* (Fares F A et al., Proc Natl Acad. Sci. USA, 1992, 89: 4304-4308, 1992).

The term "glycosylation" means that an oligosaccharide (a carbohydrate containing two or more monosaccharides that are linked together, e.g., a carbohydrate containing 2 to about 12 monosaccharides that are linked together) is attached to form a glycoprotein. The oligosaccharide side chains are generally linked to the backbone of the glycoprotein via N- or *O*-linkages. The oligosaccharides of the antibodies disclosed herein are generally CH2 domains linked to the Fc region as *N*-linked oligosaccharides. "*N*-linked glycosylation" refers to carbohydrate moiety attachment to an asparagine residue of a glycoprotein chain. For example, the skilled artisan can recognize a single site useful for *N*-linked glycosylation at residue 297 of each of CH2 domains of murine IgG1, IgG2a, IgG2b and IgG3 and human IgG1, IgG2, IgG3, IgG4, IgA and IgD.

The term "conservative modification" is intended to mean that an amino acid modification does not significantly affect or change the binding characteristics of the antibody containing an amino acid sequence. Such conservative modifications include amino acid substitutions, additions and deletions. A modification may be introduced into the antibody of the present disclosure by using a standard technology known in the art, such as a site-directed mutagenesis and a PCR-mediated mutagenesis. A conservative amino acid substitution refers to the substitution of an amino acid residue with an amino acid residue with a similar side chain. Families of amino acid residues with similar side chains have been described in detail in the art. These families include amino acids with basic side chains (such as lysine, arginine and histidine), amino acids with acidic side chains (such as aspartic acid and glutamic acid), amino acids with uncharged polar side chains (such as glycine, asparagine, glutamine, serine, threonine, tyrosine, cysteine and tryptophan), amino acids with non-polar side chains (such as alanine, valine, leucine, isoleucine, proline, phenylalanine and methionine), amino acids with β-branched side chains (such as threonine, valine and isoleucine) and amino acids with aromatic side chains (such as tyrosine, phenylalanine, tryptophan and histidine). Therefore, one or more amino acid residues in the CDR of the antibody of the present disclosure may be substituted with other amino acid residues from the same side chain family.

### Fc variant with altered binding affinity for the neonatal receptor (FcRn)

"FcRn" used herein refers to a protein that binds to at least part of the Fc region of the IgG antibody and that is encoded by the FcRn gene. FcRn may be derived from any organism including, but not limited to, humans, mice, rats, rabbits or monkeys. The functional FcRn protein includes two polypeptides that often referred to as heavy and light chains, in which the light chain is β-2-microglobulin and the heavy chain is encoded by the FcRn gene.

The present disclosure relates to an antibody whose binding to FcRn is regulated (the regulation includes to increase or decrease the binding). For example, in some cases, increased binding may result in cell recirculating antibodies, and thus extends, for example, the half-life of the therapeutic antibody. Sometimes, it is desirable to decrease the FcRn binding, for example, when the antibody is used as a diagnostic or therapeutic antibody including a radiolabel. In addition, antibodies exhibiting increased binding to FcRn and altered binding to other Fc receptors such as Fcγ Rs may be used in the present disclosure.

The present application relates to an antibody including an amino acid modification that regulates the binding to FcRn. Of particular interest is that at lower pH, the binding affinity for FcRn exhibits an increase, while at higher pH, the binding basically does not exhibit an altered antibody that minimally includes the Fc region or functional variants thereof.

### Fc variant with enhanced binding affinity for the neonatal receptor (FcRn)

The plasma half-life of IgG depends on its binding to FcRn, where IgG generally binds to FcRn at a pH of 6.0 and dissociates from FcRn at a pH of 7.4 (the pH of plasma). Through studies on the binding site, a binding site of IgG to FcRn is modified to increase the binding capacity at the pH of 6.0. It has been proved that mutations of some residues of a human Fcγ domain, which are essential to the binding to FcRn, can increase the serum half-life. It has been reported that mutations at T250, M252, S254, T256, V308, E380, M428, and N434 (EU numbering) can increase or decrease the binding affinity for FcRn (Roopenian et al., Nat. Rev. Immunol., 7: 715-725, 2007). Korean Patent No. KR 10-1027427 discloses trastuzumab (Herceptin, Genentech) variants with enhanced binding affinity for FcRn, where these variants include one or more amino acid modifications selected from 257C, 257M, 257L, 257N, 257Y, 279Q, 279Y, 308F, and 308Y. Korean Patent Publication No. KR 2010-0099179 provides bevacizumab (Avastin, Genentech) variants, where these variants exhibit an increased *in vivo* half-life through amino acid modifications included in N434S, M252Y/M428L, M252Y/N434S, and M428L/N434S. In addition, Hinton et al. have found that T250Q and M428L mutants increase the binding to FcRn threefold and sevenfold, respectively. At the same time, the mutation of two sites increases the binding 28-fold. In rhesus monkeys, the M428L or T250QM/428 L mutant exhibits the plasma half-life increased twofold (Hinton P.R. et al., J. Immunol., 176: 346-356, 2006). For more mutational sites included in the Fc variant with the enhanced binding affinity for the neonatal receptor (FcRn), see Chinese invention patent CN 201280066663.2. In addition, studies have shown that through the T250Q/M428L mutation on Fc fragments of five humanized antibodies, the interaction between Fc and FcRn is improved, and in subsequent *in vivo* pharmacokinetic assays, the pharmacokinetic parameters of the Fc-mutation antibody are improved compared with the wild-type antibody through subcutaneous administration, for example, the *in vivo* exposure is increased, the clearance rate is reduced, and the subcutaneous bioavailability is increased (Datta-Mannan A et al., MAbs. Taylor&Francis, 4: 267-273, 2012).

Other mutational sites capable of enhancing the affinity of the antibody of the present disclosure for FcRn include, but are not limited to, the following amino acid modifications: 226, 227, 230, 233, 239, 241, 243, 246, 259, 264, 265, 267, 269, 270, 276, 284, 285, 288, 289, 290, 291, 292, 294, 298, 299, 301, 302, 303, 305, 307, 309, 311, 315, 317, 320, 322, 325, 327, 330, 332, 334, 335, 338, 340, 342, 343, 345, 347, 350, 352, 354, 355, 356, 359, 360, 361, 362, 369, 370, 371, 375, 378, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 433, 438, 439, 440, 443, 444, 445, and 446, where the numbers of the amino acids in the Fc region is numbers of the EU indexes in Kabat.

Fc variants with enhanced binding affinity for FcRn also include all other known amino acid modification sites as well as amino acid modification sites that have not yet been found.

In an optional embodiment, the IgG variant can be optimized to gain increased or decreased affinity for FcRn and increased or decreased affinity for human FcyR including, but not limited to, FcγRI, FcγRIIa, FcγRIIb, FcγRIIc, FcγRIIIa and FcγRIIIb, including allelic variants thereof.

Preferentially, the Fc ligand specificity of an IgG variant determines its therapeutic application. The given IgG variant for therapeutic purposes depends on the epitope or form of the target antigen as well as the to-be-treated disease or indication. Enhanced FcRn binding may be more preferred for most targets and indications because enhanced FcRn binding may result in extended serum half-life. A relatively long serum half-life allows administration at relatively low frequencies and doses during treatment. This property may be particularly preferred when the therapeutic agent is administered in order to respond to indications requiring repeated administration. For some targets and indications, the reduced affinity for FcRn may be particularly preferred when the variant Fc is required to have an increased clearance or reduced serum half-life, for example, when the Fc polypeptide is used as an imaging agent or a radiotherapy agent.

### Fc alterations to extend the half-life

As used herein, "Fc alterations to extend the half-life" refers to an alteration to extend the *in vivo* half-life of a protein that includes an altered Fc polypeptide in the Fc polypeptide chain as compared with the half-life of a protein that includes the same Fc polypeptide but does not include any altered but similar Fc. These alterations may be included in the Fc polypeptide chain as part of the bispecific antibody. Alterations T250Q, M252Y, S254T and T256E (alteration of threonine at position 250 to glutamine; alteration of methionine at position 252 to tyrosine; alteration of serine at position 254 to threonine; and alteration of threonine at position 256 to glutamic acid; where numbers are based on EU numbering) is an Fc alteration to extend half-life and may be used jointly, alone or in any combination. These and other alterations are described in detail in U.S. Pat. No. 7,083,784. The section about this alteration described in U.S. Pat. No. 7,083,784 is incorporated herein by reference.

Similarly, M428L and N434S are Fc alterations that extend the half-life and can be used jointly, alone or in any combination. These alterations and other alterations are described in detail in U.S. Patent Application Publication No. 2010/0234575 and U.S. Pat. No. 7,670,600. Sections of such alterations described in U.S. Patent Application Publication No. 2010/0234575 and U.S. Pat. No. 7,670,600 are incorporated herein by reference.

In addition, according to the meaning herein, any substitution at one of the following positions can be considered to be an Fc alternation that extends the half-life: 250, 251, 252, 259, 307, 308, 332, 378, 380, 428, 430, 434, and 436. Each of these alterations or a combination of these alterations may be used to extend the half-life of the bispecific antibody described herein. Other alternations that can be used to extend the half-life are described in detail in International Application No. PCT/US2012/070146 (Publication No. WO 2013/096221) filed December 17, 2012. The section about the above alterations of this application is incorporated herein by reference.

Fc alternations that extend the half-life also include sites and modifications thereof that are well known in the art or may be discovered in the future.

Fc may be derived from any organism including, but not limited to, humans, mice, rats, rabbits or monkeys.

### Nucleic acid encoding bispecific antibody

Using the therapeutic agent, the antibody or the antibody fragment described herein, those skilled in the art may easily construct multiple clones containing functionally equivalent nucleic acids (e.g., nucleic acids with different sequences but encoding the same effector moiety or antibody sequence). Therefore, the present disclosure provides bispecific antibodies, nucleic acids encoding the antibodies, antibody fragments and conjugates and fusion protein thereof, and variants, derivatives and species homologues of the nucleic acids.

Many nucleic acid sequences encoding immunoglobulin regions including VH, VL, hinge, CH1, CH2, CH3 and CH4 regions are known in the art. See, for example, Kabat et al., Sequences of Proteins of Immunological Interest, Public Health Service N.I.H., Bethesda, MD, 1991. According to the teachings provided herein, those skilled in the art may combine the nucleic acid sequences and/or other nucleic acid sequences known in the art to construct a nucleic acid sequence encoding the bispecific antibody of the present disclosure. An exemplary nucleotide encoding the bispecific antibody of the present disclosure includes SEQ ID NO: 51.

In addition, based on the amino acid sequences provided herein and elsewhere and the common knowledge in the art, those skilled in the art may determine the nucleic acid sequence encoding the bispecific antibody of the present disclosure. In addition to more conventional methods for producing cloned DNA fragments encoding particular amino acid sequences, companies such as DNA 2.0 (Menlo Park, CA, USA) and Blue Heron (Bothell, WA, USA) generally employ chemical synthesis to produce DNA that has any size of genes arranged in a desired order, thus simplifying the process of producing the DNA.

### Method for preparing a bispecific antibody

The bispecific antibody of the present disclosure may be prepared by any method known in the art. Early methods for constructing the bispecific antibody include chemical cross-linking or hybridoma heterozygosis or quadroma method (e.g., Staerz UD et al., Nature, 314: 628-31, 1985; Milstein C et al., Nature, 305: 537-540, 1983; Karpovsky B et al., J. Exp. Med., 160: 1686-1701, 1984). The chemical coupling method is to connect two different monoclonal antibodies by chemical coupling to prepare a bispecific monoclonal antibody. For example, two different monoclonal antibodies chemically bind to each other, or two antibody fragments, for example, two Fab fragments chemically bind to each other. The heterozygosis-hybridoma method is to prepare a bispecific monoclonal antibody by a cell hybridization method or a ternary hybridoma method, where the cell hybridoma or the ternary hybridoma is obtained by the fusion of constructed hybridomas or the fusion of a constructed hybridoma and lymphocytes derived from mice. Although these techniques are used to manufacture BiAb, various generation problems make such complexes difficult to use, such as the generation of mixed populations containing different combinations of antigen-binding sites, difficulties in protein expression, the need for purifying the target BiAb, low yields, and high production costs.

Recent methods utilize genetically engineered constructs that can produce a single homogeneous product of BiAb so that there is no need for thorough purification to remove unwanted by-products. Such constructs include tandem scFv, diabodies, tandem diabodies, double variable domain antibodies, and heterdimeric antibodies using the Ch1/Ck domain or DNLTM motifs (Chames & Baty, Curr. Opin. Drug. Discov. Devel., 12: 276-83, 2009; Chames & Baty, mAbs, 1: 539-47). Related purification techniques are well known.

The antibody may also be produced by cloning and expressing immunoglobulin variable region cDNAs produced from a single lymphocyte selected to produce the specific antibody using a single lymphocyte antibody method, for example, by a method described in Babcook J et al., Proc. Natl. Acad. Sci. USA. 93: 7843-7848, 1996; WO92/02551; WO2004/051268 and WO2004/106377.

Antigen polypeptides for producing, for example, antibodies for immunizing hosts or for screening, such as antibodies for phage display (or the expression on the surface of yeast cells or bacterial cells) may be prepared from genetically engineered host cells including expression systems by methods well known in the art, or they may be recycled from natural biological sources. For example, nucleic acids encoding one or two polypeptide chains of the bispecific antibody may be introduced into cultured host cells by a variety of known methods (such as transformation, transfection, electroporation and bombardment with nucleic acid-coated microparticles, etc.). In some embodiments, the nucleic acids encoding the bispecific antibody may be inserted into a vector suitable to be expressed in the host cell before introduced into the host cell. A typical vector may include sequence elements that enable the inserted nucleic acids to be expressed at RNA and protein levels.

The vector is well known in the art and many vectors are commercially available. The host cell containing the nucleic acids may be cultured under conditions that enable the cell to express the nucleic acids, and the resulting BiAb may be collected from a cell population or a culture medium. Optionally, the BiAb may be produced *in vivo,* for example, in a plant leaf (see, for example, Scheller J et al., Nature Biotechnol., 19: 573-577, 2001 and references cited therein), in a bird egg (see, for example, Zhu L et al., Nature Biotechnol., 23: 1159-1169, 2005 and references cited therein) or in mammalian milk (see, for example, Laible G et al., Reprod. Fertil. Dev., 25: 315, 2012).

Various cultured host cells that may be used include, for example, prokaryotic cells, eukaryotic cells, bacterial cells (such as *Escherichia coli* or *Bacilis stearothermophilus*), fungal cells (such as *Saccharomyces cerevisiae* or *Pichia pastoris*), insect cells (such as lepidopteran insect cells including Spodoptera frugiperda cells), or mammalian cells (such as Chinese hamster ovary (CHO) cells, NS0 cells, baby hamster kidney (BHK) cells, monkey kidney cells, Hela cells, human hepatocellular carcinoma cells or 293 cells).

The bispecific antibody may be prepared by immunizing an appropriate subject (such as a rabbit, a goat, a mouse, or other mammals including transgenic and knocked-out mammals) with an immunogenic preparation of a bispecific antigen. An appropriate immunogenic preparation may be, for example, a chemically synthesized or recombinantly expressed bispecific antigen. The preparation may further include adjuvants such as Freund's complete or incomplete adjuvants or similar immunostimulatory compounds. Furthermore, the bispecific antigen of the present disclosure may be used alone or preferably used as a conjugate with a vector protein when used for preparing antibodies, in particular by *in vivo* immunization. Such methods of enhancing an antibody response are well known in the art. Depending on different antibodies required, different animal hosts may be used for *in vivo* immunization. A host that expresses an endogenous antigen of interest itself may be used, or a host that has been caused deficient in an endogenous antigen of interest may be used.

The bispecific antibody may be prepared by a combination of the methods as described above.

The bispecific antibody molecule of the present disclosure may act as a monoclonal antibody (MAb) for each target. In some embodiments, the antibody is chimeric, humanized or fully human.

The monoclonal antibody may be prepared by any method known in the art, such as a hybridoma technique (Kohler&Milstein, Nature, 256: 495-497, 1975), a trioma technique, a human B-cell hybridoma technique (Kozbor D et al., Immunology Today, 4: 72, 1983), and an EBV-hybridoma technique (Cole SPC et al., Monoclonal Antibodies and Cancer Therapy, pp 77-96, Alan R Liss, Inc., 1985).

The bispecific antibody or a portion thereof in the present disclosure may be used for detecting any or all of these antigens (for example, in biological samples such as serum or plasma) by conventional immunological analysis methods such as an enzyme-linked immunosorbent assay (ELISA), a radioimmunoassay (RIA) or tissue immunohistochemistry. The present disclosure provides a method for detecting an antigen in a biological sample, comprising contacting a biological sample with the bispecific antibody or the antibody portion of the present disclosure that specifically recognizes the antigen, and detecting an antigen-bound antibody (or antibody portion) or a non-bound antibody (or antibody portion), so as to detect the antigen in the biological sample. The antibody is directly or indirectly labeled with a detectable substance to facilitate the detection of the bound or non-bound antibody. Suitable detectable substances include various enzymes, repair groups, fluorescent substances, luminescent substances and radioactive substances. Examples of suitable enzymes include horseradish peroxidase, alkaline phosphatase, β-galactosidase and acetylcholinesterase; examples of suitable repair group complexes include streptavidin/biotin and avidin/biotin; examples of suitable fluorescent substances include 7-hydroxycoumarin, fluorescein, fluorescein isothiocyanate, rhodamine, dichlorotriazinylamine fluorescein, dansyl chloride or phycoerythrin; an example of the luminescent substance includes 3-aminophthalhydrazide; examples of suitable radioactive substances include I¹²⁵, I¹³¹, ³⁵S or ³H.

### Pharmaceutical composition

The bispecific antibody of the present disclosure or a nucleic acid or polynucleotide encoding the antibody of the present application may be applied to the preparation of the pharmaceutical composition or a sterile composition. For example, the bispecific antibody is mixed with a pharmaceutically acceptable carrier, excipient or stabilizer. The pharmaceutical composition may include one bispecific antibody or a combination of (for example, two or more different) bispecific antibodies of the present disclosure. For example, the pharmaceutical composition of the present disclosure may include a combination of antibodies or antibody fragments (or immunoconjugates) that have complementary activity and bind to different epitopes on a target antigen. Formulations of therapeutic and diagnostic agents may be prepared by mixing the antibody with the pharmaceutically acceptable carrier, excipient or stabilizer in the form of, for example, lyophilized powder, slurry, an aqueous solution or a suspension.

The term "pharmaceutically acceptable" means that a molecule, molecule fragment or composition does not produce unfavorable, allergic or other adverse effects when properly administered to animals or humans. Specific examples of some substances that may act as the pharmaceutically acceptable carriers or components thereof include sugars (such as lactose), starch, cellulose and its derivatives, vegetable oils, gelatin, polyols (such as propylene glycol), alginic acid and the like.

In some preferred embodiments, the pharmaceutical composition of the present disclosure is used for treating, preventing or alleviating diseases including, but not limited to, cancer, lymphatic system diseases, autoimmune diseases, inflammatory diseases, infectious diseases, immunodeficiency syndromes and other related diseases or symptoms.

The bispecific antibody or the nucleic acid or polynucleotide encoding the antibody of the present application may be linked to the preceding pharmaceutically acceptable carriers or components thereof (as immune complexes) or administered separately from the preceding pharmaceutically acceptable carriers or the components thereof. In the latter case, the bispecific antibody or the nucleic acid or polynucleotide encoding the antibody of the present application may be administered before, after or together with the preceding pharmaceutically acceptable carriers or the components thereof or may be administered with other known therapies (such as anticancer therapies or radiation).

The compositions of the present disclosure may be in various forms. The forms include, for example, liquid, semi-solid and solid dosage forms such as liquid solutions (e.g. injectable and non-molten solutions), dispersants or suspension tablets, pills, powders, liposomes and suppositories. A preferred manner depends on the mode of administration and the therapeutic use. Preferred typical compositions are injectable or non-molten solutions, such as those compositions that passively immunize humans like other antibodies. The compositions of the present disclosure may be administered through various routes including oral, rectal, transmucosal, trans-intestinal, parenteral, intramuscular, subcutaneous, intradermal, intramedullary, intrathecal, direct intraventricular, intravenous, intraperitoneal, intranasal, intraocular, inhalation, insufflation, topical, skin, transdermal, or intraarterial. A preferred mode of administration is parenteral (e.g., intravenous, subcutaneous, intraperitoneal, intramuscular). In a preferred embodiment, the antibody is administered by intravenous infusion or injection. In another preferred embodiment, the antibody is injected intramuscularly or subcutaneously.

The above combination methods, treatment methods and administration methods are well known. Combination, treatment and administration methods that may be developed in the future are also included.

The pharmaceutical composition of the present disclosure may be a combination of two drugs or used in combination with an already marketed product having a similar function or a product that increases a therapeutic effect.

### Combination therapy

The present disclosure relates to uses of a combination of the bispecific antibody or nucleic acids or polynucleotides encoding the antibody of the present disclosure or immunoconjugates or pharmaceutical compositions and one or more active therapeutic agents (e.g., chemotherapeutic agents) or other prophylactic or therapeutic modes (e.g., radiation). In such combination therapies, the various active agents often have different complementary mechanisms of action, and the combination therapy may lead to synergistic effects. The combination therapy includes therapeutic agents that affect immune responses (e.g., an enhanced or activated response) and therapeutic agents that affect (e.g., inhibit or kill) tumor/cancer cells. The combination therapy may reduce the likelihood of drug-resistant cancer cells. The combination therapy may allow the dose reduction of one or more reagents to reduce or eliminate adverse effects associated with the one or more reagents. Such combination therapies may have synergistic therapeutic or prophylactic effects on underlying diseases, disorders or symptoms.

The "combination" includes therapies that can be administered separately, for example, separate formulations for individual administration (e.g., which may be provided in a kit), and therapies that can be administered together in a single formulation (i.e., "co-formulation"). In some embodiments, the bispecific antibody of the present disclosure or nucleic acids or polynucleotides encoding the antibody of the present disclosure or immunoconjugates or pharmaceutical compositions may be administered sequentially. In some embodiments, the bispecific antibody of the present disclosure or nucleic acids or polynucleotides encoding the antibody of the present disclosure or immunoconjugates or pharmaceutical compositions may be administered simultaneously. The bispecific antibody or nucleic acids or polynucleotides encoding the antibody of the present disclosure or immunoconjugates or pharmaceutical compositions may be used in any manner in combination with at least one other (active) agent.

Treatment with the bispecific antibody of the present disclosure may be combined with other treatments that are effective against the to-be-treated disease. Non-limiting examples of antibody combination therapies of the present disclosure include surgery, chemotherapy, radiation therapy, immunotherapy, gene therapy, DNA therapy, RNA therapy, nanotherapy, viral therapy, and adjuvant therapy.

Combination therapies also include all other combination therapies known in the art or developed in the future.

### BRIEF DESCRIPTION OF DRAWINGS

FIG. 1 illustrates SEC-HPLC test results of a purified sample of bispecific antibody AP062.
FIG. 2 illustrates SDS-PAGE electrophoresis results of a purified sample of bispecific antibody AP062.
FIG. 3 illustrates SDS-PAGE results of bispecific antibody AP062 in an acceleration experiment at 5 °C.
FIG. 4 illustrates SDS-PAGE results of bispecific antibody AP062 in an acceleration experiment at 25 °C.
FIG. 5 illustrates an ability, detected by flow cytometry, of bispecific antibody AP062 to bind to CD20-positive tumor cells.
FIG. 6 illustrates an ability of bispecific antibody AP062 to mediate effector cells to kill Raji-luc cells.
FIG. 7 illustrates abilities, detected by a reporter gene assay, of bispecific antibodies AP062 and AB7K7 to activate Jurkat NFATRE Luc cells.
FIG. 8 illustrates an *in vivo* anti-tumor effect of bispecific antibody AP062 in an NPG mouse model of transplanted tumor constructed by subcutaneously co-inoculating human CIK cells and human Burkkit's lymphoma Raji cells.
FIG. 9 illustrates an *in vivo* anti-tumor effect of bispecific antibody AP062 in an NPG mouse model of transplanted tumor constructed by subcutaneously co-inoculating human CIK cells and human Burkkit's lymphoma Daudi cells.
FIG. 10 illustrates changes of leukocytes and lymphocytes in normal cynomolgus monkeys administered with bispecific antibody AP062 multiple times.

### DETAILED DESCRIPTION

The present disclosure is further described through examples which should not be construed as further limitations. All drawings, all reference documents, and the contents of patents and published patent applications cited in the entire application are expressly incorporated herein by reference.

In the following examples, materials used in experiments may be purchased or prepared with reference to techniques disclosed in the existing art; materials whose sources and specifications are unidentified are commercially available; various processes and methods not described in detail are conventional methods well known in the art.

### Example 1 Construction of an expression vector of a bispecific antibody molecule

The bispecific antibody molecule constructed in the present disclosure is a tetravalent homodimer formed by two identical polypeptide chains binding to each other by an interchain disulfide bond in hinge regions of Fc fragments, wherein each polypeptide chain consists of an anti-CD20 scFv, a linker peptide L2, an anti-CD3 scFv and an Fc fragment in sequence from N-terminus to C-terminus, wherein VH and VL in the anti-CD20 scFv are linked by a linker peptide L1, and VH and VL in the anti-CD3 scFv are linked by a linker peptide L3. Some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of a first single-chain Fv against CD20 are listed in Table 1, wherein amino acid residues contained in the CDRs are defined according to a Kabat rule. The amino acid composition of the linker peptide L1 between VH and VL of the anti-CD20 scFv is (GGGGS)n, wherein n = 1, 2, 3, 4 or 5.

**Table 1 Amino acid sequences of the anti-CD20 scFv contained in the bispecific antibody and amino acid sequences of its CDRs**

| CD20 | | |
|---|---|---|
| SEQ ID NO: 1 | HCDR1 | SYNMH |
| SEQ ID NO: 2 | HCDR2 | AIYPGNGDTSYNQKFKG |
| SEQ ID NO: 3 | HCDR3 | STYYGGDWYFNV |
| SEQ ID NO: 4 | LCDR1 | RASSSVSYIH |
| SEQ ID NO: 5 | LCDR2 | A TSNLAS |
| SEQ ID NO: 6 | LCDR3 | QQWTSNPPT |
| SEQ ID NO: 25 | VH | |
| SEQ ID NO: 26 | VL | |

| CD20 | | |
|---|---|---|
| SEQ ID NO: 7 | HCDR1 | NYYIH |
| SEQ ID NO: 8 | HCDR2 | WIYPGDGNTKYNEKFKG |
| SEQ ID NO: 9 | HCDR3 | DSYSNYYFDY |
| SEQ ID NO: 10 | LCDR1 | RASSSVSYMH |
| SEQ ID NO: 11 | LCDR2 | APSNLAS |
| SEQ ID NO: 12 | LCDR3 | QQWSFNPPT |
| SEQ ID NO: 27 | VH | EVQLVQSGAEVKKPGASVKVSCKASGYTFTNYYIHW VRQAPGQGLEWIGWIYPGDGNTKYNEKFKGRATLT ADTSTSTAYLELSSLRSEDTAVYYCARDSYSNYYFD YWGQGTLVTVSS |
| SEQ ID NO: 28 | VL | |

| CD20 | | |
|---|---|---|
| SEQ ID NO: 13 | HCDR1 | YSWIN |
| SEQ ID NO: 14 | HCDR2 | RIFPGDGDTDYNGKFKG |
| SEQ ID NO: 15 | HCDR3 | NVFDGYWLVY |
| SEQ ID NO: 16 | LCDR1 | RSSKSLLHSNGITYLY |
| SEQ ID NO: 17 | LCDR2 | QMSNLVS |
| SEQ ID NO: 18 | LCDR3 | AQNLELPYT |
| SEQ ID NO: 29 | VH | QVQLVQSGAEVKKPGSSVKVSCKASGYAFSYSWIN WVRQAPGQGLEWMGRIFPGDGDTDYNGKFKGRVTI TADKSTSTAYMELSSLRSEDTAVYYCARNVFDGYWL VYWGQGTLVTVSS |
| SEQ ID NO: 30 | VL | DIVMTQTPLSLPVTPGEPASISCRSSKSLLHSNGITYL YWYLQKPGQSPQLLIYQMSNLVSGVPDRFSGSGSG TDFTLKISRVEAEDVGVYYCAQNLELPYTFGGGTKV EIK |

| CD20 | | |
|---|---|---|
| SEQ ID NO: 19 | HCDR1 | DYAMH |
| SEQ ID NO: 20 | HCDR2 | TISWNSGSIGYADSVKG |
| SEQ ID NO: 21 | HCDR3 | DIQYGNYYYGMDV |
| SEQ ID NO: 22 | LCDR1 | RASQSVSSYLA |
| SEQ ID NO: 23 | LCDR2 | DASNRAT |
| SEQ ID NO: 24 | LCDR3 | QQRSNWPIT |
| SEQ ID NO: 31 | VH | EVQLVESGGGLVQPGRSLRLSCAASGFTFNDYAMH |
| | | WVRQAPGKGLEWVSTISWNSGSIGYADSVKGRFTIS RDNAKKSLYLQMNSLRAEDTALYYCAKDIQYGNYYY GMDVWGQGTTVTVSS |
| SEQ ID NO: 32 | VL | EIVLTQSPATLSLSPGERATLSCRASQSVSSYLAWY QQKPGQAPRLLIYDASNRATGIPARFSGSGSGTDFT LTISSLEPEDFAVYYCQQRSNWPITFGQGTRLEIK |

The anti-CD3 scFv binds to an effector cell at an EC₅₀ value greater than about 50 nM, or greater than 100 nM, or greater than 300 nM, or greater than 500nM in an *in vitro* FACS binding assay; more preferably, the second single-chain Fv of the bispecific antibody is capable of binding to human CD3 and specifically binding to CD3 of a cynomolgus monkey or a rhesus monkey.

Some preferred amino acid sequences of the VH domain and its complementarity determining regions (HCDR1, HCDR2 and HCDR3) and amino acid sequences of the VL domain and its complementarity determining regions (LCDR1, LCDR2 and LCDR3) of the anti-CD3 scFv are listed in Table 2, wherein amino acid residues contained in the CDRs are defined according to the Kabat rule. The amino acid composition of the linker peptide L3 between VH and VL of the anti-CD3 scFv is (GGGGS)n, wherein n = 1, 2, 3, 4 or 5.

**Table 2 Amino acid sequences of the anti-CD3 scFv contained in the bispecific antibody and amino acid sequences of its CDRs**

| CD3-3 | | |
|---|---|---|
| SEQ ID NO: 34 | HCDR1 | TYAMN |
| SEQ ID NO: 35 | HCDR2 | RIRSKYNNYATYYADSVKD |
| SEQ ID NO: 36 | HCDR3 | HGNFGNSYVSWFAY |
| SEQ ID NO: 37 | LCDR1 | RSSTGAVTTSNYAN |
| SEQ ID NO: 38 | LCDR2 | GTNKRAP |
| SEQ ID NO: 39 | LCDR3 | ALWYSNLVW |
| SEQ ID NO: 46 | VH | EVQLLESGGGLVQPGGSLKLSCAASGFTFNTYAMN WVRQAPGKGLEWVARIRSKYNNYATYYADSVKDRF TISRDDSKNTAYLQMNNLKTEDTAVYYCVRHGNFGN SYVSWFAYWGQGTLVTVSS |
| SEQ ID NO: 47 | VL | ELVVTQEPSLTVSPGGTVTLTCRSSTGAVTTSNYAN WVQQKPGQAPRGLIGGTNKRAPGTPARFSGSLLGG KAALTLSGVQPEDEAEYYCALWYSNLWVFGGGTKL TVL |

| CD3-4 | | |
|---|---|---|
| SEQ ID NO: 40 | HCDR1 | KYAMN |
| SEQ ID NO: 41 | HCDR2 | RIRSKYNNYATYYADSVKD |
| SEQ ID NO: 42 | HCDR3 | HGNFGNSYISYWAY |
| SEQ ID NO: 43 | LCDR1 | GSSTGAVTSGYYPN |
| SEQ ID NO: 44 | LCDR2 | GTKFLAP |
| SEQ ID NO: 45 | LCDR3 | ALWYSNRVW |
| SEQ ID NO: 48 | VH | EVQLLESGGGLVQPGGSLKLSCAASGFTFNKYAMN WVRQAPGKGLEWVARIRSKYNNYATYYADSVKDRF TISRDDSKNTAYLQMNNLKTEDTAVYYCVRHGNFGN SYISYWAYWGQGTLVTVSS |
| SEQ ID NO: 49 | VL | ELVVTQEPSLTVSPGGTVTLTCGSSTGAVTSGYYPN WVQQKPGQAPRGLIGGTKFLAPGTPARFSGSLLGG KAALTLSGVQPEDEAEYYCALWYSNRWVFGGGTKL TVL |

The linker peptide that links the anti-CD20 scFv to the anti-CD3 scFv consists of a flexible peptide and a rigid peptide; preferably, an amino acid composition structure of the flexible peptide has a general formula of GₓS_{y}(GGGGS)_{z}, wherein x, y and z are integers greater than or equal to 0 and x + y + z ≥ 1. The rigid peptide is derived from a full-length sequence (as shown in SEQ ID NO: 33) consisting of amino acids 118 to 145 at the carboxyl terminus of natural human chorionic gonadotropin β-subunit or a truncated fragment thereof; preferably, the composition of the CTP rigid peptide is SSSSKAPPPS (CTP¹). Some preferred amino acid sequences of the linker peptide L2 that links the anti-CD20 scFv and the anti-CD3 scFv are listed in Table 3.

**Table 3 Amino acid sequences of the linker peptide that links the anti-CD20 scFv to the anti-CD3 scFv**

| | | |
|---|---|---|
| SEQ ID NO: 52 | G2(GGGGS)3CTP1 | GGGGGGSGGGGSGGGGSSSSSKAPPPS |
| SEQ ID NO: 53 | (GGGGS)3CTP1 | GGGGSGGGGSGGGGSSSSSKAPPPS |
| SEQ ID NO: 54 | GS(GGGGS)2CTP1 | GSGGGGSGGGGSSSSSKAPPPS |
| SEQ ID NO: 55 | (GGGGS)1CTP4 | GGGGSSSSSKAPPPSLPSPSRLPGPSDTPILPQ |

The Fc fragment is linked to the anti-CD3 scFv directly or by a linker peptide, wherein the linker peptide includes 1-20 amino acids, and preferably selected from the following amino acids: Gly(G), Ser(S), Ala(A) and Thr(T), more preferably selected from Gly (G) and Ser (S), and most preferably, the linker peptide consists of (GGGGS)n, wherein n = 1, 2, 3 or 4.

The Fc fragment is preferably selected from heavy chain constant regions of human IgG1, IgG2, IgG3 and IgG4 and more particularly selected from heavy chain constant regions of human IgG1 or IgG4; and Fc is mutated to modify the properties of the bispecific antibody molecule, e.g., reduced affinity to at least one of human FcyRs (FcyRI, FcγRIIa or FcγRIIIa) and C1q, a reduced effector cell function, or a reduced complement function. In addition, the Fc fragment may also contain amino acid substitutions that change one or more other characteristics (such as an ability of binding to an FcRn receptor, the glycosylation of the antibody or the charge heterogeneity of the antibody).

Some amino acid sequences of the Fc fragment with one or more amino acid mutations are listed in Table 4.

**Table 4 Amino acid sequences of Fc from human IgG**

| **Amino acid sequence of constant region of IgG1 Fc (L234A/L235A) mutant (EU numbering)** | |
|---|---|
| SEQ ID NO: 56 | DKTHTCPPCP APEAAGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPGK |

| **Amino acid sequence of constant region of IgG1 (L234A/L235A/T250Q/N297A/P331S/M428L/K447-) mutant (EU numbering)** | |
|---|---|
| SEQ ID NO: 57 | DKTHTCPPCP APEAAGGPSV FLFPPKPKDQ LMISRTPEVT CVVVDVSHED PEVKFNWYVD GVEVHNAKTK PREEQYASTY RVVSVLTVLH QDWLNGKEYK CKVSNKALPA SIEKTISKAK GQPREPQVYT LPPSRDELTK NQVSLTCLVK GFYPSDIAVE WESNGQPENN YKTTPPVLDS DGSFFLYSKL TVDKSRWQQG NVFSCSVLHE ALHNHYTQKS LSLSPGK |

Exemplarily, the amino acid sequence of the preferred bispecific antibody AP062 is shown by SEQ ID NO: 50, and the corresponding nucleotide sequence is shown by SEQ ID NO: 51.

A gene encoding the bispecific antibody was synthesized by a conventional molecular biology method, and cDNA encoding the obtained fusion gene was separately inserted into corresponding restriction sites of a eukaryotic expression plasmid pCMAB2M modified by PCDNA3.1. The plasmid pCMAB2M also contains a selective marker to have kanamycin resistance in bacteria and G418 resistance in mammalian cells. In addition, when host cells are deficient in the expression of DHFR genes, the expression vector pCMAB2M contains mouse dihydrofolate reductase (DHFR) genes so that target genes and the DHFR genes can be co-amplified in the presence of methotrexate (MTX) (see U.S. patent No. 4399216).

### Example 2 Expression of the bispecific antibody molecule

The constructed expression plasmid was transfected into a mammalian host cell line to express the bispecific antibody. To achieve stable and high-level expression, a preferred host cell line is DHFR deficient CHO-cells (see U.S. Patent No. 4818679). In this example, a CHO-derived cell strain DXB11 was selected as the host cell. A preferred method of transfection is electroporation, and other methods, including calcium phosphate co-precipitation and lipofection, may also be used. During electroporation, 50 µg of DNA of the expression vector plasmid were added to 5×10⁷ cells in a cuvette with a Gene Pulser electroporator (Bio-Rad Laboratories, Hercules, CA) set at an electric field of 300 V and a capacitance of 1500 µFd. Two days after transfection, the medium was replaced with a growth medium containing 0.6 mg/mL G418. Transfectants were subcloned by limiting dilution and the secretion rate of each cell line was measured through an ELISA. The cell strain that expressed the bispecific antibody at a high level was screened.

To achieve the higher-level expression of fusion proteins, DHFR genes inhibited by MTX should be used for co-amplification. The transfected fusion protein genes were co-amplified with the DHFR genes in growth media containing MTX with increased concentrations. Subclones with the positive expression of DHFR were subjected to limiting dilution with gradually increased pressure to screen transfectants capable of growing in media with MTX of up to 6 µM. The secretion rates of the transfectants were determined and a cell line with high foreign protein expression was screened. A cell line with a secretion rate greater than 5 (preferably about 15) µg/10⁶ (millions) cells/24 h was adaptively suspended using a serum-free medium. Cell supernatants were collected and the bispecific antibody was separated and purified.

### Example 3 Purification process and stability test of the bispecific antibody

### 3.1 Purification of the bispecific antibody

The bispecific antibody was purified by three-step chromatography. The three-step chromatography was respectively affinity chromatography, hydroxyapatite chromatography and anion exchange chromatography. (The protein purifier used in this example was AKTA pure 25 M from GE in the U.S. Reagents used in this example were purchased from Sinopharm Chemical Reagent Co., Ltd and had purity at an analytical grade).

In a first step, affinity chromatography was performed. Sample capture and concentration and the removal of partial pollutants were performed using AT Protein A Diamond from Bestchrom or other commercially available affinity media (such as MabSelect Sure from GE). First, chromatography columns were equilibrated with 3-5 column volumes (CVs) of an equilibration buffer (20 mM PB, 150 mM NaCl, pH 7.4) at a linear flow rate of 100-200 cm/h. The clarified fermentation broth was loaded at a linear flow rate of 100-200 cm/h with a load not higher than 20 mg/mL. After loading, the chromatography columns were equilibrated with 3-5 column volumes (CVs) of an equilibration buffer (20 mM PB, 150 mM NaCl, pH 7.4) at a linear flow rate of 100-200 cm/h to remove unbound components. The chromatography columns were rinsed with 3-5 column volumes decontamination buffer 1 (50 mM NaAc-HAc, 1 M NaCl, pH 5.0) at a linear flow rate of 100-200 cm/h to remove partial pollutants. The chromatography columns were equilibrated with 3-5 column volumes (CVs) of decontamination buffer 2 (50 mM NaAc-HAc, pH 5.0) at a linear flow rate of 100-200 cm/h. The target product was eluted using an elution buffer (50 mM NaAc-HAc, pH 3.5) at a linear flow rate not higher than 100 cm/h and target peaks were collected.

In a second step, hydroxyapatite chromatography was performed. Intermediate purification was performed using CHT Type II from BIO-RAD or other commercially available hydroxyapatite media to reduce the content of polymers. After the target proteins were polymerized, the polymers and monomers differed in property such as charge characteristics and calcium ion chelation. The polymers and the monomers were separated with differences between charge characteristics. First, chromatography columns were equilibrated with 3-5 column volumes (CVs) of an equilibration buffer (20 mM PB, pH 7.4) at a linear flow rate of 100-200 cm/h. The target proteins separated through the affinity chromatography in the first step were loaded after its pH was adjusted to 7.0, with a load controlled to be less than 5 mg/mL. After loading, the chromatography columns were rinsed with 3-5 column volumes (CVs) of an equilibration buffer (20 mM PB, pH 7.0) at a linear flow rate of 100-200 cm/h. Finally, the target proteins were eluted using 10 column volumes (CVs) of an elution buffer (20 mM PB, 1M NaCl, pH 7.0) at a linear flow rate not higher than 100 cm/h with a gradient of 0-25%. Eluted fractions were collected and sent for SEC-HPLC, resepectively. Target components with the percentage of monomers being greater than 95% were combined for chromatography in the next step.

In a third step, anion exchange chromatography was performed. Fine purification was performed by using DEAE Sepharose Fast Flow from GE or other commercially available anion exchange chromatography media (such as Q HP of Bestchrom, Toyopearl GigaCap Q-650 of TOSOH, DEAE Beads 6FF of Smart-Lifesciences, Generik MC-Q of Sepax Technologies, Inc, Fractogel EMD TMAE of Merck, and Q Ceramic HyperD F of Pall) to further remove pollutants such as HCP and DNA. First, chromatography columns were rinsed with 3-5 column volumes (CVs) of an equilibration buffer (20 mM PB, 0.15 M NaCl, pH 7.0) at a linear flow rate of 100-200 cm/h. The target proteins separated through hydroxyapatite chromatography in the second stop were loaded and through-flow was collected. After loading, the chromatography columns were rinsed with 3-5 column volumes (CVs) of an equilibration buffer (20 mM PB, 0.15 M NaCl, pH 7.0) at a linear flow rate of 100-200 cm/h. The through-flow components were collected and sent for the detection of protein content, SEC-HPLC and electrophoresis.

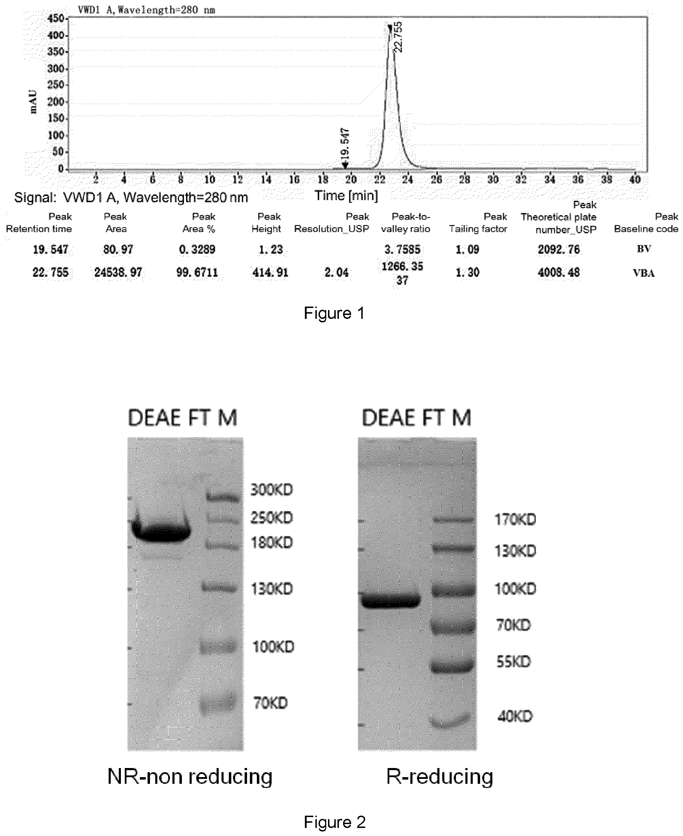

The SEC-HPLC purity results and SDS-PAGE electrophoresis results of the samples are shown in FIG. 1 and FIG. 2. The SEC-HPLC results showed that the purity of the main peak of the bispecific antibody was more than 99.0% after three-step chromatography. The band pattern in the SDS-PAGE electrophoresis was as expected, where a band was shown at 160 KDa in the non-reducing electrophoresis and a clear single-chain band (80 KDa) was obtained after reduction.

### 3.2 Stability test of the bispecific antibody

The stability of a bispecific antibody AP062 in histidine salts (20 mM histidine, 8% sucrose, 0.02% Tween-80) at different pH (pH 5.5, 6.0 and 6.5) was investigated and the effect of shaking on the stability of the sample was explored. The bispecific antibody AP062 was stored for two weeks under accelerated conditions at 5 °C and 25 °C for the evaluation of protein stability.

AP062 was transferred to histidine buffers of pH 5.5, 6.0 and 6.5, separately. Samples were taken at different time points for detection and analysis. The detection items included SEC-HPLC and SDS-PAGE.

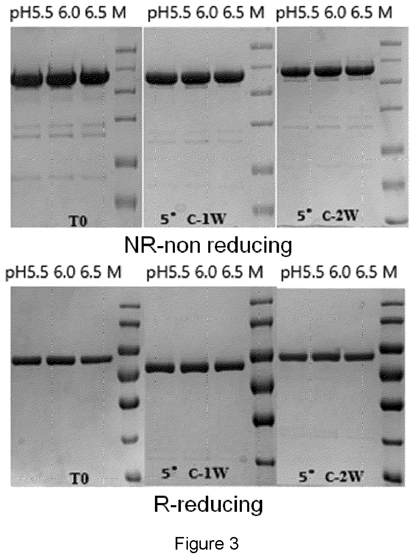

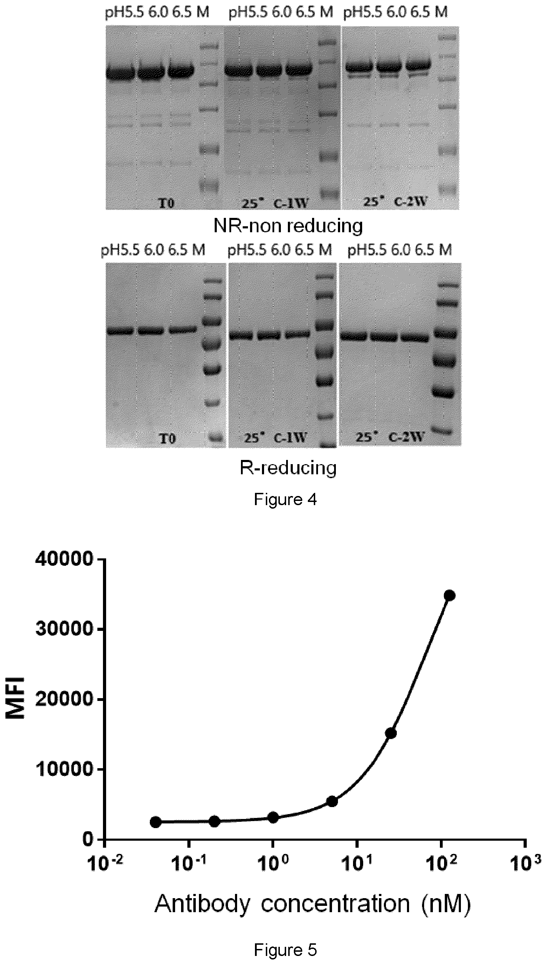

SEC-HPLC and SDS-PAGE test results of three preparations stored for 0-2 weeks at 5 °C and 25 °C are shown in Tables 5 and 6 and FIGS. 3 and 4. After the samples were placed still for two weeks, the proportions of the polymers, main peaks, shoulder peaks and fragments in the SEC-HPLC test results have no significant changes and differ little at different pH. There is no significant difference in SEC-HPLC results over two weeks in the histidine buffers of pH 5.5, 6.0 and 6.5.

**Table 5 SEC-HPLC results of acceleration experiments at 5 °C**

| | SEC-polymer % | | | SEC-main peak % | | | SEC-fragment % | | |
|---|---|---|---|---|---|---|---|---|---|
| | T0 | 1W | 2W | T0 | 1W | 2W | T0 | 1W | 2W |
| pH 5.5 | 2.5 | 1.1 | 2.7 | 95.6 | 97.4 | 96.6 | 1.8 | 1.6 | 0.7 |
| pH 6.0 | 1.9 | 0.7 | 2.8 | 96.3 | 97.9 | 96.6 | 1.8 | 1.3 | 0.7 |
| pH 6.5 | 2.3 | 0.8 | 2.9 | 95.6 | 97.4 | 96.4 | 2.1 | 1.8 | 0.7 |

**Table 6 SEC-HPLC results of acceleration experiments at 25 °C**

| | SEC-polymer% | | | SEC-main peak% | | | SEC-fragment% | | |
|---|---|---|---|---|---|---|---|---|---|
| | T0 | 1W | 2W | T0 | 1W | 2W | T0 | 1W | 2W |
| pH 5.5 | 2.5 | 0.8 | 2.1 | 95.6 | 97.5 | 97.2 | 1.8 | 1.7 | 0.8 |
| pH 6.0 | 1.9 | 0.6 | 2.4 | 96.3 | 97.7 | 96.9 | 1.8 | 1.7 | 0.7 |
| pH 6.5 | 2.3 | 0.6 | 2.2 | 95.6 | 97.6 | 97.0 | 2.1 | 1.8 | 0.9 |

### Example 4 In vitro pharmacodynamic study of the bispecific antibody

### 4.1 Detection of the binding activity of AP062 to CD20-positive tumor cells by flow cytometry

Raji cells (purchased from the cell bank of Chinese Academy of Sciences) were cultured and collected by centrifugation. The collected cells were resuspended with 1% PBSB and placed in 96-well plates, 100 µl (i.e., 2 × 10⁵ cells) per well, after the cell density was adjusted to (2 × 10⁶) cells/ml. Diluted bispecific antibodies with a series of concentrations were added and incubated for 1 hour at 4 °C. The cells were centrifuged to discard the supernatant and then washed three times using a PBS solution with 1% BSA (PBSB). Diluted AF488-labeled goat anti-human IgG antibodies (Jackson Immuno Research Inc., Cat. No. 109-545-088) or mouse anti-6xHis IgG antibodies (R&D Systems, Cat. No. IC050P) were added to the cells, and the cells were incubated for 1 hour at 4 °C in the dark. The obtained cells were centrifuged to discard the supernatant and then washed twice with 1% PBSB, and cells in each well were resuspended with 100 µl of 1% paraformaldehyde. The signal intensity was detected by flow cytometry. The analysis was performed with the average fluorescence intensity as the Y-axis and the antibody concentration as the X-axis through software GraphPad to calculate the EC₅₀ value for the binding of AP062 to Raji cells.

As shown in FIG. 5, AP062 bound well to CD20-positive cells, the signal intensity was proportional to the antibody concentration, and the EC₅₀ value for AP062 binding to Raji cells was calculated, which was about 69.97 nM.

### 4.2 AP062 mediating effector cells to target and kill CD20-positive tumor cells

Normally cultured Raji-luc cells (purchased from Beijing Biocytogen Biotechnology Co., Ltd.) were added to 96-well white plates after the cell density was adjusted to 1 × 10⁵ cells/ml, 40 µl per well. AP062 antibodies were diluted into a series of gradients and added to the 96-well white plates. After the CIK cell density was adjusted to 5 × 10⁵ cells/ml, the CIK cells were added to the 96-well white plates, 40 µl per well, to make the effector: target ratio (E : T) equal to 5 : 1, and cultured for 24 hours at 37 °C. After 24 hours, the white plates were taken out, 100 µl of One-Glo (Promega, Cat. No. E6120) solution was added to each well, and then the white plates were placed for at least three minutes at room temperature. The luminescence value was measured by a microplate reader. The analysis was performed with the fluorescence intensity as the Y-axis and the antibody concentration as the X-axis through software GraphPad to calculate the EC₅₀ value of AP062 killing Raji-luc cells.

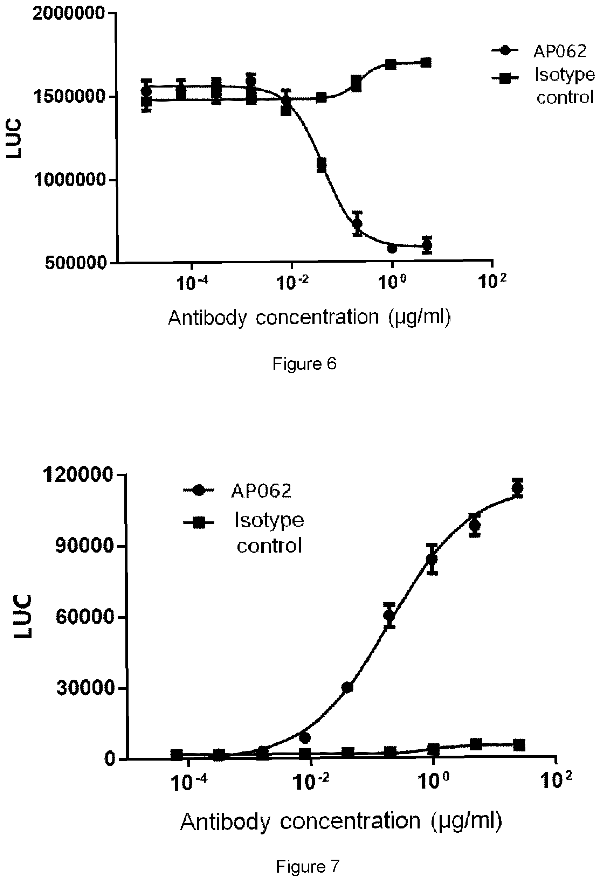

As shown in FIG. 6, the EC₅₀ for AP062 mediating effector cells to kill Raji-luc cells was only 42.8 ng/ml and AP062 had target specificity, while the EC₅₀ of isotype antibody as a negative control was 229.5 ng/ml and isotype antibody had little killing effect on Raji-luc cells.

### 4.3 Evaluation of abilities of bispecific antibodies to activate T cells through reporter gene cell strains

Jurkat T cells containing NFAT RE reporter genes (BPS Bioscience, Cat. No. 60621) can overexpress luciferase in the presence of bispecific antibodies and CD20-positive Raji cells, and the degree of activation of the Jurkat T cells can be quantified by detecting the activity of the luciferase. A four-parameter curve was fitted using the concentration of bispecific antibody as the X-axis and the fluorescein signal as the Y-axis.

As shown in FIG. 7, AP062 can specifically activate Jurkat NFATRE Luc cells, wherein the EC₅₀ value was 0.2006 µg/ml and its concentration was proportional to signal intensity, while AB7K7 as a negative control had little ability to activate T cells.

### Example 5 In vivo pharmacodynamic study of the bispecific antibody

### 5.1 NPG mouse model of transplanted tumor established by co-inoculating subcutaneously human CIK cells and human Burkkit's lymphoma Raji cells

Human Burkkit's lymphoma Raji cells with positive CD20 expression were selected to observe the *in vivo* anti-tumor effect of the bispecific antibody in a NPG mouse model of transplanted tumor that was established by co-inoculating subcutaneously human CIK cells and human Burkkit's lymphoma Raji cells.

The peripheral blood of a normal human was taken. Human PBMCs were separated through density gradient centrifugation (Lymphoprep™, human lymphocyte isolation solution, STEMCELL), then re-suspended in a RPMI-1640 culture medium added with 10% inactivated FBS, and added with OKT3 at a final concentration of 1 µg/mL and human IL-2 at 250 IU/mL. After three days of culture, the mixture was centrifuged at 300 g for 5 min, and the medium was replaced with RPMI-1640 added with 10% inactivated FBS for cell culture and human IL-2 at 250 IU/mL. A fresh medium was then added every 2 days, and CIK cells were collected on the tenth day of culture. Female NPG mice at the age of seven to eight weeks (purchased from Beijing Vitalstar Biotechnology Co., Ltd.) were selected and Raji cells in the logarithmic growth stage were collected. 4×10⁶ Raji cells and 8×10⁵ CIK cells were mixed and inoculated subcutaneously on the right back of each NPG mouse. One hour later, the mice were randomly divided into 5 groups with 6 mice in each group according to their weight. They were intraperitoneally administered with corresponding drugs. All administration groups were administered twice per week. The positive control monoclonal antibody Rituxan (Merlot, Roche Pharmaceuticals) and the bispecific antibody AP062 wereseparately administered at a dose of 1 mg/kg and a dose of 0.1 mg/kg. The day of administration was recorded as Day 0. The maximum diameter (D) and the minimum diameter (d) of the tumor were measured twice per week. The volume of the tumor was calculated using the following formula: volume (mm³) = [D×d²]/2. The tumor growth inhibition rate was calculated for each administration group using the following formula: TGI (%) = (1 - the volume of the administration group/the volume of the control group) × 100%.

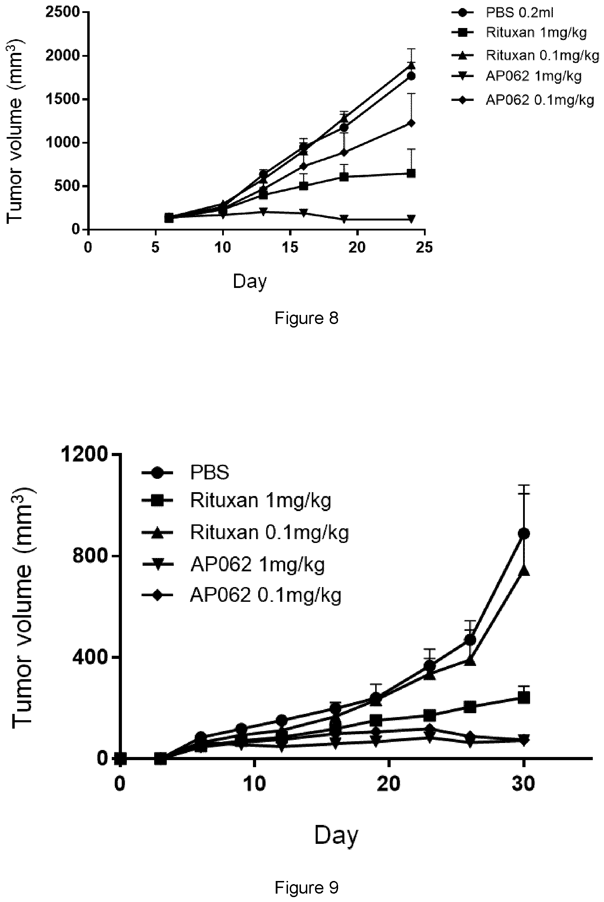

As shown in FIG. 8, on Day 24 of administration, the average tumor volume of the PBS control group was 1766.84 ± 155.62 mm³; the average tumor volume of the treated group administrated with Rituxan at a dose of 1 mg/kg was 647.92 ± 277.11 mm³, and TGI was 63.33%, which was significantly different from that of the control group (*P* < *0.01*); the average tumor volume of the treated group administrated with Rituxan at a dose of 0.1 mg/kg was 1893.81 ± 186.99 mm³, and Rituxan herein exhibited no efficacy; the average tumor volume of the treated group administrated with AP062 at a dose of 1 mg/kg was 116.18 ± 39.50 mm³, and TGI was 93.42%, which was significantly different from that of the control group (*P* < *0.01*); the average tumor volume of the treated group administrated with AP062 at a dose of 0.1 mg/kg was 1226.03 ± 340.05 mm³, and TGI was 30.61%, which was not significantly different from that of the control group. The results show that the bispecific antibody AP062 could inhibit the growth of tumor cells by activating human immune cells in animals; and at the same dose, the efficacy of the bispecific antibody was better than the efficacy of the monoclonal antibody Rituxan, and the bispecific antibody exhibited great anti-tumor effects.

### 5.2 NPG mouse model of transplanted tumor constructed by subcutaneously co-inoculating human CIK cells and human Burkkit's lymphoma Daudi cells

CD20-positive human Burkkit's lymphoma Daudi cells were selected to study the inhibiting effect of bispecific antibodies on tumor growth *in vivo* in an NPG mouse model of transplanted tumor constructed by subcutaneously co-inoculating human CIK cells and human Burkkit's lymphoma Daudi cells.

CIK cells were prepared in the method as described in Example 5.1. Female NPG mice at the age of seven to eight weeks were selected, and Daudi cells in the logarithmic growth stage were collected. 4 × 10⁶ Daudi cells and 8 × 10⁵ CIK cells were mixed and inoculated subcutaneously on the right back of each NPG mouse. One hour later, the mice were randomly divided into five groups with six mice in each group according to their weights and intraperitoneally administered with corresponding drugs. All treated groups were administrated twice a week. Rituxan and bispecific antibody AP062 were both administered at doses of 1 mg/kg and 0.1 mg/kg, respectively. The day of administration was recorded as Day 0. The maximum diameter (D) and the minimum diameter (d) of the tumor were measured weekly. The volume (mm³) of the tumor of each group and the tumor growth inhibition rate (TGI) (%) of each treated group were calculated using the formulas as shown in Example 5.1.

As shown in FIG. 9, on Day 30 of administration, the average tumor volume of the PBS control group was 889.68 ± 192.13 mm³; the average tumor volume of the treated group administrated with Rituxan at a dose of 1 mg/kg was 241.51 ± 44.91 mm³, and TGI was 72.85%, which was significantly different from that of the control group (*P* < *0.01*); the average tumor volume of the treated group administrated with Rituxan at a dose of 0.1 mg/kg was 746.11 ± 299.71 mm³, which was not significantly different from that of the control group; the average tumor volume of the treated group administrated with AP062 at a dose of 1 mg/kg was 72.05 ± 11.89 mm³, and TGI was 91.9%, which was significantly different from that of the control group (*P* < *0.01*); the average tumor volume of the treated group administrated with AP062 at a dose of 0.1 mg/kg was 75.36 ± 11.81 mm³, and TGI was 91.53%, which was significantly different from that of the control group (*P* < *0.01*). The results show that the bispecific antibody AP062 could inhibit the growth of tumor cells by activating human immune cells in animals; and at the same dose, the efficacy of the bispecific antibody was better than the efficacy of the monoclonal antibody Rituxan, and AP062 exhibited good anti-tumor effects even at a low dose.

### Example 6 Evaluation of the safety of bispecific antibodies

The toxicity of AP062 was evaluated to determine appropriate dose ranges and observation indicators for subsequent toxicity tests. Adult Female cynomolgus monkeys (purchased from Guangzhou Xiangguan Biotechnology Co., Ltd.) at the age of 3-4 years and with the weight of 3-4 kg were divided into two groups with one mouse in each group, wherein the two groups were a vehicle control group and an AP062 treated group. The groups were administrated via intravenous drip by a peristaltic pump for 1 hour. The dose amount and volume administered are shown in Table 7. The groups were administrated on Day 0 (D0), Day 7 (D7), Day 21 (D21), and Day 28 (D28), respectively, for a total of four doses, and the drug dose was gradually escalated each time. The monkeys were weighed weekly.

**Table 7 Dosing schedule for cynomolgus monkey acute toxicity evaluation**

| Grou p | To-be-tested drugs name | Dose volume | Dose amount |
|---|---|---|---|
| G1 | Vehicle control group | D0: 5 mL/kg | N/A |
| | | D7: 5 mL/kg | |
| | | D21: 10 mL/kg | |
| | | D28: 10 mL/kg | |
| G2 | AP062 | D0: 5 mL/kg | D0: 0.06 mg/kg |
| | | D7: 5 mL/kg | D7: 0.3 mg/kg |
| | | D21: 10 mL/kg | D21: 1.5 mg/kg |
| | | D28: 10 mL/kg | D28: 3 mg/kg |

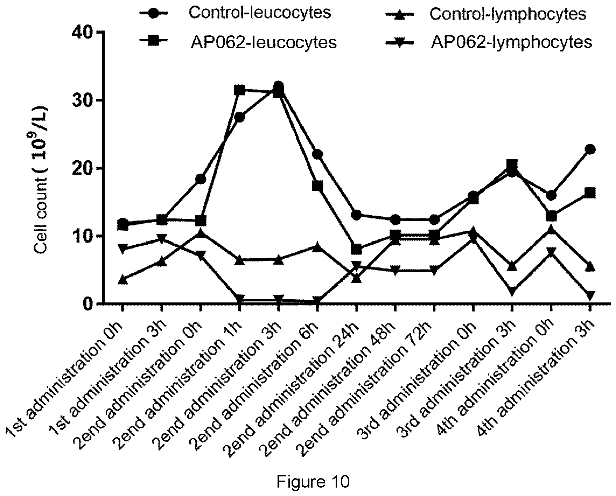

During the test, animals were periodically monitored for clinical symptoms, body weight, food consumption, body temperature, electrocardiogram, blood pressure, clinicopathological indexes (blood cell count, coagulation function measure, and blood biochemistry), lymphocyte subsets, cytokines, drug plasma concentration measure, and toxicokinetics analyses. After administration of AP062, the physical signs of cynomolgus monkeys exhibited no abnormal reaction, the body weight was relatively stable, the body temperature fluctuation was similar to the body temperature fluctuation of the vehicle control group, and no death or impending death was observed among animals during the administration period. As shown in FIG. 10, after administration, the white blood cell changes of cynomolgus monkeys in the AP062 group were similar to the white blood cell changes in the control group; the first administration of AP062 at a dose of 0.06 mg/kg had little effect on lymphocytes; 1 hour to 6 hours after the second administration, the number of lymphocytes in the animals of the treated group decreased sharply and recovered to normal after 24 hours; as the number of administrations increased, the effect of AP062 on the decrease in the number of lymphocytes was weaker and weaker despite increasing doses. In addition, after the first administration of AP062, the release of IL-2, IL-6 and TNF-α factors was promoted and the release of IL-5 was slightly stimulated, but the release of IFN-γ was not stimulated; as the number of administrations increased, the release-promoting effect of AP062 on cytokines became less and less significant, indicating that the body had already been adapted to the stimulation by bispecific antibodies.

### Example 7 Pharmacokinetics evaluation of Anti-CD20xCD3 bispecific antibodies

Female cynomolgus monkeys with the weight of 3-4 kg were divided into two groups with one in one monkey in each group. The first group was a blank control group, and the second group was an AP062 treated group administrated at a dose of 0.3 mg/kg. The blood sampling time points were Minute 15, Hour 1, Hour 3, Hour 6, Hour 10, Hour 24, Hour 30, Hour 48, Hour 54, Hour 72, Hour 96, and Hour 144, respectively, a total of 13 time points. Serum was collected from blood and frozen at -80°C.

The drug concentration of AP062 in serum was determined by ELISA. The pharmacokinetics parameters were calculated using software PKSolver. Specific parameters are shown in Table 8. The results show that T_{1/2} of AP062 in normal cynomolgus monkeys was about 8.5 hours.

**Table 8 Pharmacokinetics parameters of bispecific antibody AP062 in cynomolgus monkeys**

| AP062 | t_{1/2} (h) | AUC 0-inf_obs (µg/mL*h) | Vz_obs (µg/kg)/(µg/mL) | Cl_obs (µg/kg)/(µg/mL)/h |
|---|---|---|---|---|
| Pharmacokin etics parameter | 8.45 | 168.63 | 21.68 | 1.78 |

Though the preferred examples of the present disclosure are illustrated and described, it should be understood that those skilled in the art may make various changes in accordance with the teachings herein within the scope of the present disclosure.

All the publications mentioned in the present disclosure are incorporated herein by reference as if each publication is separately incorporated herein by reference. In addition, it should be understood that those skilled in the art, who have read the preceding content of the present disclosure, may make various changes or modifications on the present disclosure, and these equivalent forms fall within the scope of the appended claims.

## Claims

1. A bispecific antibody, which is a tetravalent homodimer formed by two identical polypeptide chains that bind to each other by a covalent bond, wherein each of the polypeptide chains comprises a first single-chain Fv that specifically binds to tumor antigen CD20, a second single-chain Fv that specifically binds to effector cell antigen CD3 and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions comprising CDC, ADCC and ADCP.

2. The bispecific antibody according to claim 1, wherein the first single-chain Fv comprises a VH domain and a VL domain that are linked by a linker peptide which has an amino acid sequence of (GGGGX)n, wherein X comprises Ser or Ala, preferably Ser, and n is a natural number from 1 to 5, preferably 3.

3. The bispecific antibody according to claim 1, wherein the first single-chain Fv is selected from the group consisting of:
(1) a VH domain comprising HCDR1, HCDR2 and HCDR3 as shown in SEQ ID NOs: 1, 2 and 3, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 1, 2 and 3; and a VL domain comprising LCDR1, LCDR2 and LCDR3 as shown in SEQ ID NOs: 4, 5 and 6, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 4, 5 and 6;
(2) a VH domain comprising HCDR1, HCDR2 and HCDR3 as shown in SEQ ID NOs: 7, 8 and 9, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 7, 8 and 9; and a VL domain comprising LCDR1, LCDR2 and LCDR3 as shown in SEQ ID NOs: 10, 11 and 12, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 10, 11 and 12;
(3) a VH domain comprising HCDR1, HCDR2 and HCDR3 as shown in SEQ ID NOs: 13, 14 and 15, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 13, 14 and 15; and a VL domain comprising LCDR1, LCDR2 and LCDR3 as shown in SEQ ID NOs: 16, 17 and 18, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 16, 17 and 18; and
(4) a VH domain comprising HCDR1, HCDR2 and HCDR3 as shown in SEQ ID NOs: 19, 20 and 21, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 19, 20 and 21; and a VL domain comprising LCDR1, LCDR2 and LCDR3 as shown in SEQ ID NOs: 22, 23 and 24, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 22, 23 and 24.

4. The bispecific antibody according to claim 1 or 3, wherein the first single-chain Fv is selected from the group consisting of:
(1) a VH domain comprising an amino acid sequence as shown in SEQ ID NO: 25 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 25; and a VL domain comprising an amino acid sequence as shown in SEQ ID NO: 26 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 26;
(2) a VH domain comprising an amino acid sequence as shown in SEQ ID NO: 27 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 27; and a VL domain comprising an amino acid sequence as shown in SEQ ID NO: 28 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 28;
(3) a VH domain comprising an amino acid sequence as shown in SEQ ID NO: 29 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 29; and a VL domain comprising an amino acid sequence as shown in SEQ ID NO: 30 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 30; and
(4) a VH domain comprising an amino acid sequence as shown in SEQ ID NO: 31 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 31; and a VL domain comprising an amino acid sequence as shown in SEQ ID NO: 32 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 32.

5. The bispecific antibody according to claim 1, wherein the second single-chain Fv comprises a VH domain and a VL domain that are linked by a linker peptide which has an amino acid sequence of (GGGGX)ₙ, wherein X comprises Ser or Ala, preferably Ser, and n is a natural number from 1 to 5, preferably 3.

6. The bispecific antibody according to claim 1 or 5, wherein the second single-chain Fv binds to an effector cell at an EC₅₀ value greater than about 50 nM, or greater than 100 nM, or greater than 300 nM, or greater than 500 nM in an *in vitro* binding affinity assay; more preferably, the second single-chain Fv of the bispecific antibody is capable of binding to human CD3 and specifically binding to CD3 of a cynomolgus monkey or a rhesus monkey.

7. The bispecific antibody according to claim 6, wherein the second single-chain Fv is selected from the group consisting of:
(1) a VH domain comprising HCDR1, HCDR2 and HCDR3 as shown in SEQ ID NOs: 34, 35 and 36, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 34, 35 and 36; and a VL domain comprising LCDR1, LCDR2 and LCDR3 as shown in SEQ ID NOs: 37, 38 and 39, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 37, 38 and 39; and
(2) a VH domain comprising HCDR1, HCDR2 and HCDR3 as shown in SEQ ID NOs: 40, 41 and 42, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 40, 41 and 42; and a VL domain comprising LCDR1, LCDR2 and LCDR3 as shown in SEQ ID NOs: 43, 44 and 45, respectively or having sequences that are substantially identical to (for example, are at least 80%, 85%, 90%, 92%, 95%, 97%, 98%, 99% or more similar to or have one or more amino acid substitutions (such as conservative substitutions) than) any of SEQ ID NOs: 43, 44 and 45.

8. The bispecific antibody according to claim 7, wherein the second single-chain Fv is selected from the group consisting of:
(1) a VH domain comprising an amino acid sequence as shown in SEQ ID NO: 46 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 43; and a VL domain comprising an amino acid sequence as shown in SEQ ID NO: 47 or having a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 47; and
(2) a second single-chain Fv that specifically binds to CD3; wherein the VH domain thereof contains an amino acid sequence as shown in SEQ ID NO: 48 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 48; and the VL domain thereof contains an amino acid sequence as shown in SEQ ID NO: 49 or has a sequence that is substantially identical to (for example, is at least 80%, 85%, 90%, 92%, 95%, 97%, 98%, 99% or more similar to or has one or more amino acid substitutions (such as conservative substitutions) than) SEQ ID NO: 49.

9. The bispecific antibody according to claim 1, wherein the linker peptide that links the first single-chain Fv to the second single-chain Fv consists of a flexible peptide and a rigid peptide; wherein the flexible peptide comprises two or more amino acids, and preferably selected from the following amino acids: Gly(G), Ser(S), Ala(A) and Thr(T); more preferably, the flexible peptide comprises G and S residues; most preferably, an amino acid composition structure of the flexible peptide has a general formula of GₓS_{y}(GGGGS)_{z}, wherein x, y and z are integers greater than or equal to 0 and x + y + z ≥ 1; the rigid peptide is derived from a full-length sequence consisting of amino acids 118 to 145 at carboxyl terminus of natural human chorionic gonadotropin β-subunit or a truncated fragment thereof; preferably, the rigid peptide comprises SSSSKAPPPS.

10. The bispecific antibody according to claim 9, wherein the linker peptide contains an amino acid sequence as shown in SEQ ID NO: 52.

11. The bispecific antibody according to claim 1, wherein the linker peptide that links the Fc fragment to the second single-chain Fv comprises 1-20 amino acids, and preferably selected from the following amino acids: Gly(G), Ser(S), Ala(A) and Thr(T); more preferably selected from Gly (G) and Ser (S); further preferably, the linker peptide consists of (GGGGS)n, wherein n = 1, 2, 3 or 4.

12. The bispecific antibody according to claim 1 or 11, wherein the Fc fragment comprises a hinge region, a CH2 domain and a CH3 domain derived from a human immunoglobulin heavy chain constant region; preferably, the Fc fragment is selected from heavy chain constant regions of human IgG1, IgG2, IgG3, IgG4, IgM, IgA1, IgA2, IgD and IgE; more preferably, the Fc fragment is selected from heavy chain constant regions of human IgG1, IgG2, IgG3 and IgG4; further preferably, the Fc fragment is selected from a heavy chain constant region of human IgG1 or IgG4; and the Fc fragment has one or more amino acid substitutions, deletions or additions than a natural sequence from which the Fc fragment is derived.

13. The bispecific antibody according to claim 12, wherein the Fc fragment comprises amino acid substitutions, deletions or additions with reduced or eliminated effector functions (ADCP, ADCC and CDC effects).

14. The bispecific antibody according to claim 13, wherein the Fc fragment comprises amino acid substitutions L234A/L235A/P331S that are determined according to an EU numbering system.

15. The bispecific antibody according to claim 13 or 14, wherein the Fc fragment further comprises amino acid substitutions, deletions or additions with one or more of the following properties:
(1) enhanced binding affinity to a neonatal receptor (FcRn);
(2) reduced or eliminated glycosylation; and
(3) reduced or eliminated charge heterogeneity.

16. The bispecific antibody according to claim 15, wherein the Fc fragment further comprises one or more of amino acid substitutions, deletions or additions as follow:
(1) amino acid substitutions M428L, T250Q/M428L, M428L/N434S or M252Y/S254T/T256E determined according to the EU numbering system;
(2) an amino acid substitution N297A determined according to the EU numbering system; and
(3) an amino acid deletion K447 determined according to the EU numbering system.

17. The bispecific antibody according to claim 15, wherein the Fc fragment has an amino acid sequence as shown in SEQ ID NO: 57 that has six amino acid substitutions or replacements L234A/L235A/N297A/P331S/T250Q/M428L determined according to the EU numbering system and a deleted or removed K447 determined according to the EU numbering system compared to the natural sequence from which the Fc fragment is derived.

18. The bispecific antibody according to claim 1, wherein the bispecific antibody binds to human CD20 and CD3 and has an amino acid sequence as follows:
(1) a sequence as shown in SEQ ID NO: 50;
(2) a sequence having one or more substitutions, deletions or additions (such as 1, 2, 3, 4 or 5 substitutions, deletions or additions) relative to the sequence as shown in SEQ ID NO: 50; or
(3) a sequence having at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 50.

19. A DNA molecule encoding the bispecific antibody according to any one of claims 1 to 18.

20. The DNA molecule according to claim 19, which has a nucleotide sequence as shown in SEQ ID NO: 51.

21. A vector, comprising the DNA molecule according to claim 19 or 20.

22. A host cell, comprising the vector according to claim 21, wherein the host cell comprises a prokaryotic cell, a yeast or a mammalian cell, preferably a CHO cell.

23. A pharmaceutical composition, comprising the bispecific antibody according to any one of claims 1 to 18 and a pharmaceutically acceptable excipient, carrier or diluent.

24. A method for preparing the bispecific antibody according to any one of claims 1 to 18, comprising: (a) obtaining a fusion gene of the bispecific antibody, and constructing an expression vector of the bispecific antibody; (b) transfecting the expression vector into a host cell by a genetic engineering method; (c) culturing the host cell under conditions that allow the bispecific antibody to be generated; (d) separating and purifying the bispecific antibody;
wherein the expression vector in step (a) is one or more selected from a plasmid, a bacterium and a virus; preferably, the expression vector is a pCDNA3.4 vector;
wherein the host cell into which the constructed vector is transfected by a genetic engineering method in step (b) comprises a prokaryotic cell, a yeast or a mammalian cell, such as a CHO cell, an NS0 cell or another mammalian cell, preferably a CHO cell; and
wherein the bispecific antibody is separated and purified in step (d) by a conventional immunoglobulin purification method comprising protein A affinity chromatography and ion exchange, hydrophobic chromatography or molecular sieve chromatography.

25. Use of the bispecific antibody according to any one of claims 1 to 18 for preparing a drug for treating, preventing or alleviating a tumor, wherein examples of the tumor comprise, but are not limited to, acute myeloid leukemia (AML), chronic myeloid leukemia (CML), B acute lymphocytic leukemia (B-ALL), B chronic lymphocytic leukemia (B-CLL), B-cell lymphoma (BCL), T-cell lymphoma (TCL) (such as skin), myelodysplastic syndrome (MDS), small lymphocytic lymphoma (SLL), hairy cell leukemia (HCL), marginal zone lymphoma (MZL) (such as extranodal or splenic), follicular lymphoma (FL) (such as pediatric or gastrointestinal), B-cell prolymphocytic leukemia (B-PLL), mantle cell lymphoma (MCL), lymphoplasmacytic lymphoma (LPL)/Waldenstrom's macroglobulinemia (WM), lymphoblastic leukemia (ALL) (such as B cell), lymphoblastic lymphoma (LBL) (such as B cell), plasmablastic lymphoma (PBL) (such as B cell), Hodgkin's lymphoma, non-Hodgkin's lymphoma, diffuse large B-cell lymphoma (DLBCL) (for example, primary or inflammation-related), Burkitt's lymphoma (BL), multiple myeloma, anaplastic large-cell lymphoma, HIV-related lymphoma and Waldenstrom's macroglobulinemia.

26. Use of the bispecific antibody according to any one of claims 1 to 18 for preparing a drug for treating, preventing or alleviating an autoimmune or inflammatory disease, wherein the autoimmune or inflammatory disease is selected from rheumatoid arthritis (RA), osteoarthritis, reactive arthritis, systemic lupus erythematosus (SLE), Crohn's disease, multiple sclerosis, scleroderma, psoriasis, psoriatic arthritis, ulcerative colitis (such as chronic), insulin-dependent diabetes (such as juvenile), thyroiditis (such as chronic), hyperthyroidism, asthma, allergic diseases, sarcoidosis, autoimmune hemolytic anemia, pernicious anemia, graft-versus-host disease, dermatomyositis, chronic hepatitis, microscopic renal vasculitis, chronic active hepatitis, uveitis, intestinal synovitis, autoimmune intestinal disease, idiopathic leukopenia, autoimmune glomerulonephritis, autoimmune hemolytic anemia, autoimmune hepatitis, interstitial pneumonia, chronic pemphigus, pemphigus vulgaris, arteritis, polyarteritis nodosa and ankylosing spondylitis.

27. The bispecific antibody according to any one of claims 1 to 18 or the pharmaceutical composition according to claim 23 for use in a method for enhancing or stimulating an immune response or function, comprising administering a therapeutically effective amount of the bispecific antibody or the pharmaceutical composition to an individual.

28. The bispecific antibody according to any one of claims 1 to 18 or the pharmaceutical composition according to claim 23 for use in method for preventing/treating, delaying development of, or reducing/inhibiting recurrence of a disease including diseases or disorders comprising an immune-related disease, a tumor, an autoimmune disease, an inflammatory disease or a transplant rejection-related disease or disorder, comprising administering an effective amount of the bispecific antibody or the pharmaceutical composition to an individual suffering from the diseases or disorders.


SEQUENCE LISTING

<110>  AMPSOURCE BIOPHARMA SHANGHAI INC.

<120>  BISPECIFIC ANTIBODY BINDING TO CD20 AND CD3 AND USES THEREOF

<130>  A4658EP

<140>  PCT/CN2019/113930
<141>  2019-10-29

<150>  CN201811294887.4
<151>  2018-11-01

<160>  57

<170>  PatentIn version 3.5

<210>  1
<211>  5
<212>  PRT
<213>  Artificial Sequence

<220>
<223>  anti-CD20 antibody 1 HCDR1 amino acid sequence

<400>  1

Ser Tyr Asn Met His
1               5

<210>  2
<211>  17
<212>  PRT
<213>  Artificial Sequence

<220>
<223>  anti-CD20 antibody 1 HCDR2 amino acid sequence

<400>  2

Ala Ile Tyr Pro Gly Asn Gly Asp Thr Ser Tyr Asn Gln Lys Phe Lys
1               5                   10                  15

Gly

<210>  3
<211>  12
<212>  PRT
<213>  Artificial Sequence

<220>
<223>  anti-CD20 antibody 1 HCDR3 amino acid sequence

<400>  3

Ser Thr Tyr Tyr Gly Gly Asp Trp Tyr Phe Asn Val
1               5                   10

<210>  4
<211>  10
<212>  PRT
<213>  Artificial Sequence

<220>
<223>  anti-CD20 antibody 1 LCDR1 amino acid sequence

<400>  4

Arg Ala Ser Ser Ser Val Ser Tyr Ile His
1               5                   10


<210>  5
<211>  7
<212>  PRT
<213>  Artificial Sequence

<220>
<223>  anti-CD20 antibody 1 LCDR2 amino acid sequence

<400>  5

Ala Thr Ser Asn Leu Ala Ser
1               5


<210>  6
<211>  9
<212>  PRT
<213>  Artificial Sequence

<220>
<223>  anti-CD20 antibody 1 LCDR3 amino acid sequence

<400>  6

Gln Gln Trp Thr Ser Asn Pro Pro Thr
1               5


<210>  7
<211>  5
<212>  PRT
<213>  Artificial Sequence

<220>
<223>  anti-CD20 antibody 2 HCDR1 amino acid sequence

<400>  7

Asn Tyr Tyr Ile His
1               5


<210>  8
<211>  17
<212>  PRT
<213>  Artificial Sequence

<220>

<223>  anti-CD20 antibody 2 HCDR2 amino acid sequence

<400>  8

Trp Ile Tyr Pro Gly Asp Gly Asn Thr Lys Tyr Asn Glu Lys Phe Lys
1               5                   10                  15

Gly

<210>  9
<211>  10
<212>  PRT
<213>  Artificial Sequence

<220>
<223>  anti-CD20 antibody 2 HCDR3 amino acid sequence

<400>  9

Asp Ser Tyr Ser Asn Tyr Tyr Phe Asp Tyr
1               5                   10

<210>  10
<211>  10
<212>  PRT
<213>  Artificial Sequence

<220>
<223>  anti-CD20 antibody 2 LCDR1 amino acid sequence

<400>  10

Arg Ala Ser Ser Ser Val Ser Tyr Met His
1               5                   10

<210>  11
<211>  7
<212>  PRT
<213>  Artificial Sequence

<220>
<223>  anti-CD20 antibody 2 LCDR2 amino acid sequence

<400>  11

Ala Pro Ser Asn Leu Ala Ser
1               5

<210>  12
<211>  9
<212>  PRT
<213>  Artificial Sequence

<220>
<223>  anti-CD20 antibody 2 LCDR3 amino acid sequence

<400>  12

Gln Gln Trp Ser Phe Asn Pro Pro Thr
1               5

<210>  13
<211>  5
<212>  PRT
<213>  Artificial Sequence

<220>
<223>  anti-CD20 antibody 3 HCDR1 amino acid sequence

<400>  13

Tyr Ser Trp Ile Asn
1               5

<210>  14
<211>  17
<212>  PRT
<213>  Artificial Sequence

<220>
<223>  anti-CD20 antibody 3 HCDR2 amino acid sequence

<400>  14

Arg Ile Phe Pro Gly Asp Gly Asp Thr Asp Tyr Asn Gly Lys Phe Lys
1               5                   10                  15

Gly

<210>  15
<211>  10
<212>  PRT
<213>  Artificial Sequence

<220>
<223>  anti-CD20 antibody 3 HCDR3 amino acid sequence

<400>  15

Asn Val Phe Asp Gly Tyr Trp Leu Val Tyr
1               5                   10

<210>  16
<211>  16
<212>  PRT
<213>  Artificial Sequence

<220>
<223>  anti-CD20 antibody 3 LCDR1 amino acid sequence

<400>  16

Arg Ser Ser Lys Ser Leu Leu His Ser Asn Gly Ile Thr Tyr Leu Tyr
1               5                   10                  15

<210>  17
<211>  7
<212>  PRT
<213>  Artificial Sequence

<220>
<223>  anti-CD20 antibody 3 LCDR2 amino acid sequence

<400>  17

Gln Met Ser Asn Leu Val Ser
1               5


<210>  18
<211>  9
<212>  PRT
<213>  Artificial Sequence

<220>
<223>  anti-CD20 antibody 3 LCDR3 amino acid sequence

<400>  18

Ala Gln Asn Leu Glu Leu Pro Tyr Thr
1               5


<210>  19
<211>  5
<212>  PRT
<213>  Artificial Sequence

<220>
<223>  anti-CD20 antibody 4 HCDR1 amino acid sequence

<400>  19

Asp Tyr Ala Met His
1               5


<210>  20
<211>  17
<212>  PRT
<213>  Artificial Sequence

<220>
<223>  anti-CD20 antibody 4 HCDR2 amino acid sequence

<400>  20

Thr Ile Ser Trp Asn Ser Gly Ser Ile Gly Tyr Ala Asp Ser Val Lys
1               5                   10                  15


Gly


<210>  21

<211>  13
<212>  PRT
<213>  Artificial Sequence

<220>
<223>  anti-CD20 antibody 4 HCDR3 amino acid sequence

<400>  21

Asp Ile Gln Tyr Gly Asn Tyr Tyr Tyr Gly Met Asp Val
1               5                   10


<210>  22
<211>  11
<212>  PRT
<213>  Artificial Sequence

<220>
<223>  anti-CD20 antibody 4 LCDR1 amino acid sequence

<400>  22

Arg Ala Ser Gln Ser Val Ser Ser Tyr Leu Ala
1               5                   10


<210>  23
<211>  7
<212>  PRT
<213>  Artificial Sequence

<220>
<223>  anti-CD20 antibody 4 LCDR2 amino acid sequence

<400>  23

Asp Ala Ser Asn Arg Ala Thr
1               5


<210>  24
<211>  9
<212>  PRT
<213>  Artificial Sequence

<220>
<223>  anti-CD20 antibody 4 LCDR3 amino acid sequence

<400>  24

Gln Gln Arg Ser Asn Trp Pro Ile Thr
1               5


<210>  25
<211>  121
<212>  PRT
<213>  Artificial Sequence

<220>
<223>  anti-CD20 antibody 1 VH amino acid sequence

<400>  25


<210>  26
<211>  106
<212>  PRT
<213>  Artificial Sequence

<220>
<223>  anti-CD20 antibody 1 VL amino acid sequence

<400>  26

<210>  27
<211>  119
<212>  PRT
<213>  Artificial Sequence

<220>
<223>  anti-CD20 antibody 2 VH amino acid sequence

<400>  27

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Asn Thr Lys Tyr Asn Glu Lys Phe
    50                  55                  60

Lys Gly Arg Ala Thr Leu Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Ser Tyr Ser Asn Tyr Tyr Phe Asp Tyr Trp Gly Gln Gly
            100                 105                 110

Thr Leu Val Thr Val Ser Ser
        115

<210>  28
<211>  106
<212>  PRT
<213>  Artificial Sequence

<220>
<223>  anti-CD20 antibody 2 VL amino acid sequence

<400>  28

<210> 29
<211> 119
<212> PRT
<213> Artificial Sequence

<220>
<223> anti-CD20 antibody 3 VH amino acid sequence

<400> 29

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
        115

<210>  30
<211>  112
<212>  PRT
<213>  Artificial Sequence

<220>
<223>  anti-CD20 antibody 3 VL amino acid sequence

<400>  30

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

<210>  31
<211>  122
<212>  PRT
<213>  Artificial Sequence

<220>
<223>  anti-CD20 antibody 4 VH amino acid sequence

<400>  31

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser
        115                 120

<210>  32
<211>  107
<212>  PRT
<213>  Artificial Sequence

<220>
<223>  anti-CD20 antibody 4 VL amino acid sequence

<400>  32

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Ile
                85                  90                  95

Thr Phe Gly Gln Gly Thr Arg Leu Glu Ile Lys
            100                 105

<210>  33
<211>  28
<212>  PRT

<213>  Homo sapiens

<400>  33

Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25

<210>  34
<211>  5
<212>  PRT
<213>  Artificial Sequence

<220>
<223>  anti-CD3 antibody 3 HCDR1 amino acid sequence

<400>  34

Thr Tyr Ala Met Asn
1               5

<210>  35
<211>  19
<212>  PRT
<213>  Artificial Sequence

<220>
<223>  anti-CD3 antibody 3 HCDR2 amino acid sequence

<400>  35

Arg Ile Arg Ser Lys Tyr Asn Asn Tyr Ala Thr Tyr Tyr Ala Asp Ser
1               5                   10                  15

Val Lys Asp

<210>  36
<211>  14
<212>  PRT
<213>  Artificial Sequence

<220>
<223>  anti-CD3 antibody 3 HCDR3 amino acid sequence

<400>  36

His Gly Asn Phe Gly Asn Ser Tyr Val Ser Trp Phe Ala Tyr
1               5                   10

<210>  37
<211>  14
<212>  PRT
<213>  Artificial Sequence

<220>
<223>  anti-CD3 antibody 3 LCDR1 amino acid sequence

<400>  37

Arg Ser Ser Thr Gly Ala Val Thr Thr Ser Asn Tyr Ala Asn
1               5                   10


<210>  38
<211>  7
<212>  PRT
<213>  Artificial Sequence

<220>
<223>  anti-CD3 antibody 3 LCDR2 amino acid sequence

<400>  38

Gly Thr Asn Lys Arg Ala Pro
1               5


<210>  39
<211>  9
<212>  PRT
<213>  Artificial Sequence

<220>
<223>  anti-CD3 antibody 3 LCDR3 amino acid sequence

<400>  39

Ala Leu Trp Tyr Ser Asn Leu Trp Val
1               5


<210>  40
<211>  5
<212>  PRT
<213>  Artificial Sequence

<220>
<223>  anti-CD3 antibody 4 HCDR1 amino acid sequence

<400>  40

Lys Tyr Ala Met Asn
1               5


<210>  41
<211>  19
<212>  PRT
<213>  Artificial Sequence

<220>
<223>  anti-CD3 antibody 4 HCDR2 amino acid sequence

<400>  41

Arg Ile Arg Ser Lys Tyr Asn Asn Tyr Ala Thr Tyr Tyr Ala Asp Ser
1               5                   10                  15

Val Lys Asp

<210> 42
<211> 14
<212> PRT
<213> Artificial Sequence

<220>
<223> anti-CD3 antibody 4 HCDR3 amino acid sequence

<400> 42

His Gly Asn Phe Gly Asn Ser Tyr Ile Ser Tyr Trp Ala Tyr
1               5                   10

<210> 43
<211> 14
<212> PRT
<213> Artificial Sequence

<220>
<223> anti-CD3 antibody 4 LCDR1 amino acid sequence

<400> 43

Gly Ser Ser Thr Gly Ala Val Thr Ser Gly Tyr Tyr Pro Asn
1               5                   10

<210> 44
<211> 7
<212> PRT
<213> Artificial Sequence

<220>
<223> anti-CD3 antibody 4 LCDR2 amino acid sequence

<400> 44

Gly Thr Lys Phe Leu Ala Pro
1               5

<210> 45
<211> 9
<212> PRT
<213> Artificial Sequence

<220>
<223> anti-CD3 antibody 4 LCDR3 amino acid sequence

<400> 45

Ala Leu Trp Tyr Ser Asn Arg Trp Val
1               5

<210> 46

<211>  125
<212>  PRT
<213>  Artificial Sequence

<220>
<223>  anti-CD3 antibody 3 VH amino acid sequence

<400>  46

Glu Val Gln Leu Leu Glu Ser Gly Gly Gly Leu Val Gln Pro Gly Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125


<210>  47
<211>  109
<212>  PRT
<213>  Artificial Sequence

<220>
<223>  anti-CD3 antibody 3 VL amino acid sequence

<400>  47

Glu Leu Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105

<210>  48
<211>  125
<212>  PRT
<213>  Artificial Sequence

<220>
<223>  anti-CD3 antibody 4 VH amino acid sequence

<400>  48

Glu Val Gln Leu Leu Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210>  49
<211>  109
<212>  PRT
<213>  Artificial Sequence

<220>
<223>  anti-CD3 antibody 4 VL amino acid sequence

<400>  49

Glu Leu Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Tyr Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Arg Trp Val Phe Gly Gly Gly Thr Lys Leu Thr Val Leu
            100                 105

<210>  50
<211>  744
<212>  PRT
<213>  Artificial Sequence

<220>
<223>  AP062 amino acid sequence

<400>  50

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ala Gly Gly Gly Gly Ser Gly Gly
        115                 120                 125

Gly Gly Ser Gly Gly Gly Gly Ser Gln Ile Val Leu Ser Gln Ser Pro
    130                 135                 140

Ala Ile Leu Ser Ala Ser Pro Gly Glu Lys Val Thr Met Thr Cys Arg
145                 150                 155                 160

Ala Ser Ser Ser Val Ser Tyr Ile His Trp Phe Gln Gln Lys Pro Gly
                165                 170                 175

Ser Ser Pro Lys Pro Trp Ile Tyr Ala Thr Ser Asn Leu Ala Ser Gly
            180                 185                 190

Val Pro Val Arg Phe Ser Gly Ser Gly Ser Gly Thr Ser Tyr Ser Leu
        195                 200                 205

Thr Ile Ser Arg Val Glu Ala Glu Asp Ala Ala Thr Tyr Tyr Cys Gln
    210                 215                 220

Gln Trp Thr Ser Asn Pro Pro Thr Phe Gly Gly Gly Thr Lys Leu Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
                325                 330                 335

Asp Arg Phe Thr Ile Ser Arg Asp Asp Ser Lys Asn Thr Ala Tyr Leu
            340                 345                 350

Gln Met Asn Asn Leu Lys Thr Glu Asp Thr Ala Val Tyr Tyr Cys Val
        355                 360                 365

Arg His Gly Asn Phe Gly Asn Ser Tyr Val Ser Trp Phe Ala Tyr Trp
    370                 375                 380

Gly Gln Gly Thr Leu Val Thr Val Ser Ser Gly Gly Gly Gly Ser Gly
385                 390                 395                 400

Gly Gly Gly Ser Gly Gly Gly Gly Ser Glu Leu Val Val Thr Gln Glu
                405                 410                 415

Pro Ser Leu Thr Val Ser Pro Gly Gly Thr Val Thr Leu Thr Cys Arg
            420                 425                 430

Ser Ser Thr Gly Ala Val Thr Thr Ser Asn Tyr Ala Asn Trp Val Gln
        435                 440                 445

Gln Lys Pro Gly Gln Ala Pro Arg Gly Leu Ile Gly Gly Thr Asn Lys
    450                 455                 460

Arg Ala Pro Gly Thr Pro Ala Arg Phe Ser Gly Ser Leu Leu Gly Gly
465                 470                 475                 480

Lys Ala Ala Leu Thr Leu Ser Gly Val Gln Pro Glu Asp Glu Ala Glu
                485                 490                 495

Tyr Tyr Cys Ala Leu Trp Tyr Ser Asn Leu Trp Val Phe Gly Gly Gly
            500                 505                 510

Thr Lys Leu Thr Val Leu Asp Lys Thr His Thr Cys Pro Pro Cys Pro
        515                 520                 525

Ala Pro Glu Ala Ala Gly Gly Pro Ser Val Phe Leu Phe Pro Pro Lys
    530                 535                 540

Pro Lys Asp Gln Leu Met Ile Ser Arg Thr Pro Glu Val Thr Cys Val
545                 550                 555                 560

Val Val Asp Val Ser His Glu Asp Pro Glu Val Lys Phe Asn Trp Tyr
                565                 570                 575

Val Asp Gly Val Glu Val His Asn Ala Lys Thr Lys Pro Arg Glu Glu
            580                 585                 590

Gln Tyr Ala Ser Thr Tyr Arg Val Val Ser Val Leu Thr Val Leu His
        595                 600                 605

Gln Asp Trp Leu Asn Gly Lys Glu Tyr Lys Cys Lys Val Ser Asn Lys
    610                 615                 620

Ala Leu Pro Ala Ser Ile Glu Lys Thr Ile Ser Lys Ala Lys Gly Gln
625                 630                 635                 640

Pro Arg Glu Pro Gln Val Tyr Thr Leu Pro Pro Ser Arg Asp Glu Leu
                645                 650                 655

Thr Lys Asn Gln Val Ser Leu Thr Cys Leu Val Lys Gly Phe Tyr Pro
            660                 665                 670

Ser Asp Ile Ala Val Glu Trp Glu Ser Asn Gly Gln Pro Glu Asn Asn
        675                 680                 685

Tyr Lys Thr Thr Pro Pro Val Leu Asp Ser Asp Gly Ser Phe Phe Leu
    690                 695                 700

Tyr Ser Lys Leu Thr Val Asp Lys Ser Arg Trp Gln Gln Gly Asn Val
705                 710                 715                 720

Phe Ser Cys Ser Val Leu His Glu Ala Leu His Asn His Tyr Thr Gln
                725                 730                 735

Lys Ser Leu Ser Leu Ser Pro Gly
            740

<210>  51
<211>  2232
<212>  DNA
<213>  Artificial Sequence

<220>
<223>  AP062 nucleotide sequence

<400>  51
caagtgcagc tgcagcagcc tggagctgag ctggtgaaac ccggcgcctc cgtcaagatg      60

tcctgtaagg cctccggata caccttcacc agctacaaca tgcactgggt gaaacagacc     120

cctggaaggg gcctggagtg gatcggcgcc atttaccctg gcaacggcga tacctcctat     180

aatcagaagt ttaagggcaa ggccaccctg accgctgata agagcagcag cacagcctac     240

atgcagctgt ccagcctgac ctccgaggac agcgccgtgt actactgtgc tcggagcacc     300

tactacggcg gcgactggta ctttaacgtg tggggagctg gcaccacagt gaccgtgagc     360

gccggaggag gaggatctgg aggaggaggc tccggaggag gaggaagcca gatcgtgctg 420

agccagagcc ccgccatcct gagcgccagc cccggcgaga aggtgaccat gacctgccgc 480

gccagcagca gcgtgagcta catccactgg ttccagcaga agcccggcag cagccccaag 540

ccctggatct acgccaccag caacctggcc agcggcgtgc ccgtgcgctt cagcggcagc 600

ggcagcggca ccagctacag cctgaccatc agccgcgtgg aggccgagga cgccgccacc 660

tactactgcc agcagtggac cagcaacccc cccaccttcg gcggcggcac caagctggag 720

atcaagggtg gcggcggtgg aggatccggc ggtggaggta gcggcggagg cggtagctcc 780

agctctagta aagctccccc tccttccgag gtgcagctgc tggagtccgg aggaggactg 840

gtgcagccag gaggctccct gaagctgagc tgtgctgcct ctggctttac cttcaacaca 900

tatgccatga attgggtgcg gcaggctcca ggcaagggac tggagtgggt ggctaggatc 960

aggtctaagt acaacaatta tgccacctac tatgctgatt ccgtgaagga caggttcacc 1020

atctcccgcg acgatagcaa gaacacagcc tacctgcaga tgaacaatct gaagaccgag 1080

gataccgccg tgtactactg cgtgagacat ggcaactttg gcaatagcta cgtgtcctgg 1140

ttcgcttact ggggacaggg caccctggtc acagtgagct ctggaggagg aggatctgga 1200

ggaggaggct ccggaggagg aggaagcgag ctggtggtga cccaggagcc atctctgaca 1260

gtgtcccccg gcggcacagt gaccctgaca tgtagatcca gcaccggcgc cgtgaccaca 1320

tccaactacg ctaattgggt gcagcagaag ccaggacagg ctccaagggg actgatcgga 1380

ggaaccaaca agagggctcc tggaacacca gctcggttta gcggatctct gctgggaggc 1440

aaggctgccc tgaccctgtc cggagtgcag ccagaggatg aggccgagta ttattgcgct 1500

ctgtggtata gcaatctgtg ggtgttcgga ggaggaacca agctgacagt gctggacaag 1560

acccatacat gcccaccatg ccctgcccct gaagccgccg gaggaccttc cgtgttcctg 1620

ttccctccca agccaaaaga tcagctgatg atctctagaa cccccgaagt cacctgcgtg 1680

gtcgtcgacg tgtcccatga ggaccctgaa gtcaagttca actggtacgt ggacggtgtc 1740

gaagtccaca acgccaagac caagcctagg gaggagcagt atgccagcac ataccgggtg 1800

gtgtctgtgc tgaccgtgct gcatcaggat tggctgaatg gcaaggaata taaatgtaag 1860

gtgagcaata aggctctgcc ggctagcatt gaaaaaacca tttccaaggc taagggccag 1920

cccagggagc ctcaggtcta caccctgcct ccatctagag atgaactgac caaaaaccag 1980

gtgagcctga cttgcctggt caaaggcttc taccccagcg acattgccgt ggagtgggag 2040

tctaatggcc agcctgaaaa taactacaaa actacccctc ctgtgctgga ctctgatggc 2100

tccttctttc tgtactctaa actgaccgtg gacaagtctc gctggcagca gggtaacgtg 2160

ttttcttgct ccgtgctgca cgaggctctg cataaccatt acacccagaa gagcctgtct 2220

ctgtccccag ga 2232

<210>  52
<211>  27
<212>  PRT
<213>  Artificial Sequence

<220>
<223>  G2(GGGGS)3CTP1 linker peptide

<400>  52

Gly Gly Gly Gly Gly Gly Ser Gly Gly Gly Gly Ser Gly Gly Gly Gly
1               5                   10                  15

Ser Ser Ser Ser Ser Lys Ala Pro Pro Pro Ser
            20                  25

<210>  53
<211>  25
<212>  PRT
<213>  Artificial Sequence

<220>
<223>  (GGGGS)3CTP1 linker peptide

<400>  53

Gly Gly Gly Gly Ser Gly Gly Gly Gly Ser Gly Gly Gly Gly Ser Ser
1               5                   10                  15

Ser Ser Ser Lys Ala Pro Pro Pro Ser
            20                  25

<210>  54
<211>  22
<212>  PRT
<213>  Artificial Sequence

<220>
<223>  GS(GGGGS)2CTP1 linker peptide

<400>  54

Gly Ser Gly Gly Gly Gly Ser Gly Gly Gly Gly Ser Ser Ser Ser Ser
1               5                   10                  15

Lys Ala Pro Pro Pro Ser
            20

<210>  55
<211>  33
<212>  PRT
<213>  Artificial Sequence

<220>
<223>  (GGGGS)1CTP4 linker peptide

<400>  55

Gly Gly Gly Gly Ser Ser Ser Ser Ser Lys Ala Pro Pro Pro Ser Leu
1               5                   10                  15

Pro Ser Pro Ser Arg Leu Pro Gly Pro Ser Asp Thr Pro Ile Leu Pro
            20                  25                  30

Gln

<210>  56
<211>  227
<212>  PRT
<213>  Artificial Sequence

<220>
<223>  IgG1 Fc(L234A,L235A) mutant constant region amino acid sequence

<400>  56

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  57
<211>  226
<212>  PRT
<213>  Artificial Sequence

<220>
<223>  IgG1(L234A,L235A, T250Q,N297A,P331S,M428L,K447-) mutant constant
       region amino acid sequence

<400>  57

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Gln Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Ala Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Ser Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Leu
        195                 200                 205

His Glu Ala Leu His Asn His Tyr Thr Gln Lys Ser Leu Ser Leu Ser
    210                 215                 220

Pro Gly
225